(19)

Europäisches Patentamt
European Patent Office
Office européen des brevets

(11) **EP 1 784 129 B1**

(12) **EUROPEAN PATENT SPECIFICATION**

(45) Date of publication and mention
of the grant of the patent:
**25.05.2011 Bulletin 2011/21**

(51) Int Cl.:
*A61B 8/00* (2006.01)    *G06K 9/00* (2006.01)
*A61B 8/08* (2006.01)

(21) Application number: **05795164.2**

(22) Date of filing: **29.08.2005**

(86) International application number:
**PCT/US2005/030799**

(87) International publication number:
**WO 2006/026605 (09.03.2006 Gazette 2006/10)**

(54) **SYSTEMS AND METHODS FOR QUANTIFICATION AND CLASSIFICATION OF FLUIDS IN HUMAN CAVITIES IN ULTRASOUND IMAGES**

SYSTEME UND VERFAHREN ZUR QUANTIFIZIERUNG UND KLASSIFIZIERUNG VON FLÜSSIGKEITEN IN MENSCHLICHEN KÖRPERHÖHLEN IN ULTRASCHALLBILDERN

SYSTEMES ET PROCEDES DE QUANTIFICATION ET DE CLASSIFICATION DE FLUIDES DE CAVITES HUMAINES DANS DES IMAGES ULTRASONORES

(84) Designated Contracting States:
**AT BE BG CH CY CZ DE DK EE ES FI FR GB GR HU IE IS IT LI LT LU LV MC NL PL PT RO SE SI SK TR**

(30) Priority: **27.08.2004 US 605391 P**

(43) Date of publication of application:
**16.05.2007 Bulletin 2007/20**

(73) Proprietor: **Verathon Inc.**
**Bothell, WA 98021 (US)**

(72) Inventors:
• **MCMORROW, Gerald**
**Kirkland, WA 98033 (US)**
• **CHALANA, Vikram**
**Mill Creek, WA 98027 (US)**
• **YUK, Jongtae**
**Redmond, WA 98052 (US)**

• **BAARTMANS, Henri**
**NL-3401 HT IJsselstein (NL)**
• **BOM, Nicolaas**
**NL-2825 NA Berkenwoude (NL)**
• **LANCEE, Charles, Theodoor**
**NL-9423 PH Hoogersmilde, (NL)**
• **MERKS, Egon, J., W.**
**NL-2611 WR Delft (NL)**

(74) Representative: **Wallis, Helen Frances Mary**
**Olswang LLP**
**90 High Holborn**
**London WC1V 6XX (GB)**

(56) References cited:
| | |
|---|---|
| **GB-A- 2 391 625** | **US-A- 5 235 985** |
| **US-A- 5 588 435** | **US-A- 6 146 330** |
| **US-A1- 2002 005 071** | **US-A1- 2002 102 023** |
| **US-A1- 2002 147 399** | **US-A1- 2004 127 796** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF INVENTION

This invention relates to ultrasound imaging of bodily tissues, bodily fluids, and fluid-filled cavities.

BACKGROUND OF THE INVENTION

Ultrasound imaging is accomplished by placing an ultrasound transducer on a selected location of a body and projecting ultrasound energy into the body. Acoustic waves reflecting from internal structures in the body are then received by the transducer and are processed to form an image of the internal structures. In a particular ultrasound method, amplitudes of selected harmonics of the returned signal are processed to form the ultrasound image. Briefly and in general terms, harmonic generation by the internal structures in the body is at least partially determined by the properties of the tissue that reflect the ultrasound energy, so that the presence of harmonics in the received echo may be used to generate useful information in the ultrasound image, as discussed in further detail in A. Bouakaz, E. Merks, C. Lancee, N. Bom, "Noninvasive Bladder Volume Measurements Based on Nonlinear Wave Distortions " Ultrasound in Medicine & Biology, 30:4, pp. 469-476.

In selected ultrasound imaging applications, it is often desirable to distinguish between a bodily fluid and an adjacent tissue, or between bodily fluids of different types, such as between blood and various other bodily fluids. For example, when a selected anatomical portion is imaged using B-mode ultrasound imaging, a bodily fluid and certain adjacent soft tissues within the selected portion may be relatively indistinguishable in the resulting image. Moreover, when blood within the selected portion and other bodily fluids are imaged using B-mode ultrasound, images may be generated that similarly fail to properly distinguish the blood from the other bodily fluids.

Accordingly, a new imaging system is needed that permits the diagnostician to easily distinguish or discriminate between different fluid compositions, or between a bodily fluid and tissue. It is further desirable to render more easily detectable in an ultrasound image any boundaries between cavities that contain fluids of different composition, and between a bodily fluid and a bodily tissue.

SUMMARY

There is provided an ultrasonography method, comprising creating a database that is representative of a fluid of a body; transmitting ultrasound pulses into a region-of-interest in a patient along a scan line projected into a patient's cavity containing a patient fluid; receiving echoes from the region of interest, and based upon the received echoes: determining fundamental and harmonic spectral peaks as a result of window function processing in a window region the corresponding echo amplitude response of the signals reflected from the back wall or other boundaries of the patient's body cavity, the window region spanning a portion of the tissue adjacent and distal to the cavity backwall interface and further spanning a portion of the cavity space along the scan line proximate to the cavity backwall interface; compiling an ultrasonic pattern of the region-of-interest; processing the pattern by comparing the region-of-interest patterns to the database; and determining a composition of the patient fluid within the region of interest of the patient based on the comparison.

There is also provided an ultrasonography system, comprising a database that is representative of a fluid of a body; an ultrasonic apparatus that is configured to transmit ultrasound pulses into a region-of-interest in a patient along a scan line projected into a patient's cavity containing a patient fluid and receive echoes from the region of interest, and based upon the received echoes: the system being configured to: determine fundamental and harmonic spectral peaks as a result of window function processing in a window the corresponding echo amplitude response of the signals reflected from the back wall or other boundaries of the patient's body cavity, the window region spanning a portion of the tissue adjacent and distal to the cavity backwall interface and further spanning a portion of the cavity space along the scan line proximate to the cavity backwall interface; compile an ultrasonic pattern of the region-of-interest; process the pattern by comparing the region-of-interest patterns to the database; and determine a composition of the patient fluid within the region of interest of the patient based on the comparison.

A selection of optional features is set out in the dependent claims.

BRIEF DESCRIPTION OF THE DRAWINGS

The embodiments of the present invention are described in detail below with reference to the following drawings.

FIGURE 1 is a side elevational view of a microprocessor-controlled transceiver according to an embodiment of the invention;

FIGURE 2 is a representation of an ultrasound scan cone emanating from the transceiver in a conic shape formed by a plurality of three-dimensional distributed scan lines;

FIGURE 3A is a representation of an ultrasound scan cone emanating from the transceiver in a conic shape formed by a rotational array of two-dimensional scan planes;

FIGURE 3B is a representation of a scan plane of the rotational array;

FIGURE 4 is a is depiction of the hand-held transceiver in use for scanning the abdominal area of a patient;

FIGURE 5 is a perspective view of the hand-held transceiver device sitting in a communication cradle;

FIGURE 5B illustrates a schematic view of an imaging system;

FIGURE 6 depicts a schematic view of a plurality of transceivers in connection with a server;

FIGURE 7 is a schematic view of a plurality of bladder wall measuring systems connected to a server over the Internet or other network;

FIGURE 8 illustrates a scan plane image with diagrammatic scan lines overlaid on the image;

FIGURE 9 is a schematic illustration of a scan line passing through a full bladder and a uterus;

FIGURE 10 is a plot of echo amplitude versus scan line depth of the FIGURE 9 schematic;

FIGURE 11 is a schematic illustration of a scan line passing through a near empty bladder and the uterus;

FIGURE 12 is a plot of echo amplitude versus scan line depth of the FIGURE 11 schematic;

FIGURE 13 is a schematic illustration of a scan line passing through body cavities and surrounding tissues;

FIGURE 14 is another spectral plot of a window function processed insonified region of FIGURE 13 for a non-pregnant female subject having homogenous uterine fluid;

FIGURE 15 is another spectral plot of a window function processed insonified region of FIGURE 13 for a non-pregnant female subject having heterogeneous uterine fluid;

FIGURE 16 is a calibration plot of harmonic power as a function of the bladder volume;

FIGURE 17 is a calibration plot of harmonic ratio as a function of blood composition in amniotic fluid;

FIGURE 18 is a method overview flowchart;

FIGURE 19 is an expansion of sub-algorithm 206 of FIGURE 18;

FIGURE 20 is a schematic representation of four surface patch elements; and

FIGURE 21 is a schematic representation of three scan lines passing through the subserosal and submucosal wall locations of an organ.

## DETAILED DESCRIPTION OF THE PREFERRED EMBODIMENTS

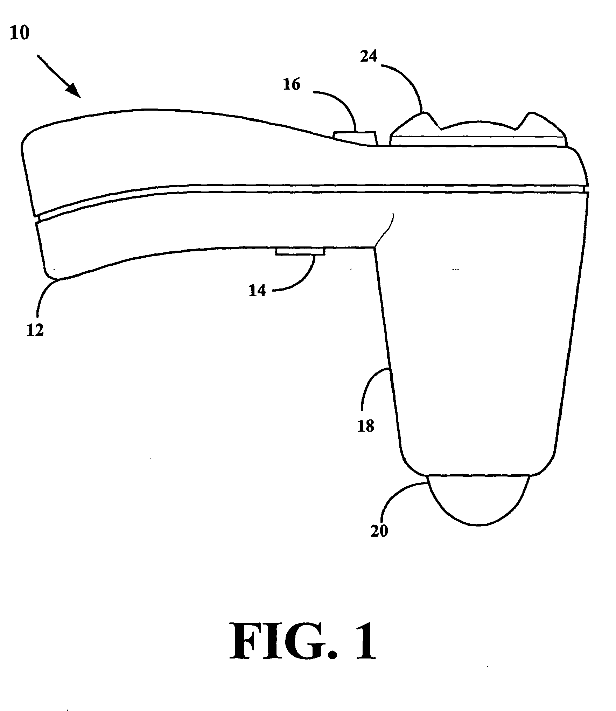

The present invention relates to the ultrasound imaging of tissues and/or fluid-filled cavities having linear or non-linear acoustic properties. Many specific details of certain embodiments of the invention are set forth in the following description and in FIGURES 1 through 21 to provide a thorough understanding of such embodiments. One skilled in the art, however, will understand that the present invention may have additional embodiments, or that the present invention may be practiced without several of the details described in the following description.

FIGURE 1 is a side elevational view of an ultrasound transceiver 10 according to an embodiment of the invention. The transceiver 10 includes a transceiver housing 18 having an outwardly extending handle 12 that is suitably configured to allow a user to manually manipulate the transceiver 10. The handle 12 includes a trigger 14 that allows the user to initiate an ultrasound scan of a selected anatomical portion, and a cavity selector 16, which will be described in greater detail below. The transceiver 10 also includes a transceiver dome 20 that contacts a surface portion of the patient when the selected anatomical portion is scanned, and a display 24 operable to view processed results from the ultrasound scan, and to allow operational interaction between the user and the transceiver 10. Accordingly, the display 24 may be configured to display alphanumeric data that indicates a proper and/or optimal position of the transceiver 10 relative to the selected anatomical portion. In other embodiments, two- or three-dimensional images of the selected anatomical region may be displayed on the display 24. The display 24 may be a liquid crystal display (LCD), a light emitting diode (LED) display, a cathode ray tube (CRT) display, or other suitable display devices operable to present alphanumeric data and/or graphical images to a user.

The transceiver 10 further includes a microprocessor (not shown in FIGURE 1) and computational algorithms (also not shown in FIGURE 1) that cooperatively provide enhanced ultrasound harmonic imaging that permits boundaries between different fluid compositions to be distinguished. In addition, the transceiver 10 may be suitably configured to distinguish between a bodily fluid and tissue, between dissimilar tissues, and/or between dissimilar bodily organs. The computational algorithms will be discussed in greater detail below. The transceiver 10 may also be coupled to a computer system (not shown in FIGURE 1) that is operable to receive either digital or analog signals from the transceiver 10 and to process the signals to generate a desired ultrasound image. In addition, the computer system may also at least partially control the operation of the transceiver 10. The computer system may comprise any microprocessor-based computer or other computer systems, such as a mainframe that is capable of executing operating instructions and manipulating data. Accordingly, the computer system is not limited to a typical desktop or laptop computer device.

The operation of the transceiver 10 will now be described. The transceiver dome 20 of the transceiver 10 is positioned against a surface portion of a patient that is proximate to the anatomical portion to be scanned. The user then actuates the transceiver 10 by depressing the trigger 14. In response, the transceiver 10 transmits ultrasound signals into the body, and receives corresponding return echo signals that are at least partially processed by the transceiver 10 to generate an ultrasound image of the selected anatomical portion. In a particular embodiment, the transceiver 10 transmits ultrasound signals in a range that extends from approximately about two megahertz (MHz) to approximately about ten MHz.

Still referring to FIGURE 1, the cavity selector 16 is structured to adjustably control the transmission and reception of ultrasound signals to the anatomy of a patient. In particular, the cavity selector 16 adapts the transceiver 10 to accommodate various anatomical details of male and female patients. For example, when the cavity selector 16 is adjusted to accommodate a male patient, the transceiver 10 is suitably configured to locate a single cavity, such as a urinary bladder in the male patient. In contrast, when the cavity selector 16 is adjusted to accommodate a female patient, the transceiver 10 is configured to image an anatomical portion having multiple cavities, such as a bodily region that includes a bladder and a uterus. Alternate embodiments of the transceiver 10 may include a cavity selector 16 that is configured to select a single cavity scanning mode, or a multiple cavity-scanning mode that may be used with male and/or female patients. The cavity selector 16 may thus permit a single cavity region to be imaged, or a multiple cavity region, such as a region that includes a lung and a heart to be imaged.

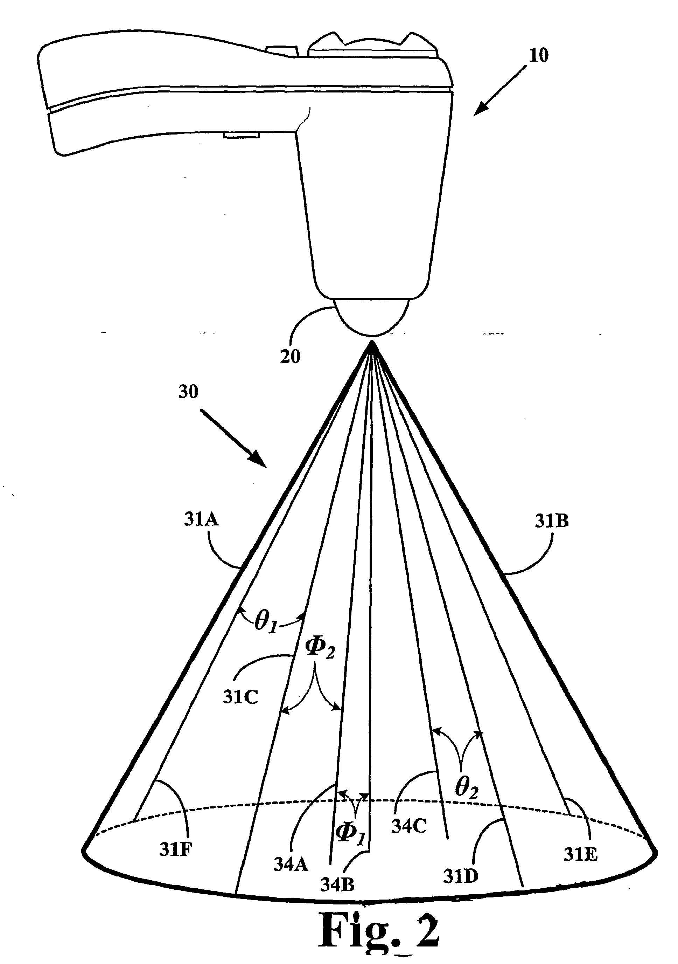

FIGURE 2 is a representation of an ultrasound scan cone 30 emanating from the transceiver 10 of FIGURE 1 that will be used to further describe the operation of the transceiver 10. The scan cone 30 has a substantially conic shape formed by a plurality of three-dimensional distributed scan lines. A scan cone 30 is shown emanating from the dome 20 of the transceiver 10 in encompassing a plurality of three-dimensional-distributed scan lines 31A-34E. The plurality of scan lines 31A-34E represent a line array in three-dimensional space. The scan lines within the line array are one-dimensional ultrasound A-lines that emanate from the transceiver 10 at different coordinate directions that taken as an aggregate form the scan cone 30. The different coordinate directions comprise a length r of a given scan line, and a rotational angle $\theta$ and a tilt angle $\phi$. Thus, one or more points P along a scan line within the line array 31A-34E are defined by the distance r and the angular coordinates $\phi$, and $\theta$.

The plurality of three-dimensional distributed scan lines 31A-34E comprises a plurality of peripheral scan lines 31A-E and a plurality of internal scan lines 34A-D. The three-dimensional-distributed A-lines (scan lines) are not necessarily confined within a scan plane, but instead are directed to sweep throughout the internal regions and along the periphery of the scan cone 30. The three-dimensional-distributed scan lines not only would occupy a given scan plane in a three-dimensional array of two-dimensional scan planes, but also the inter-scan plane spaces, from the conic axis to and including the conic periphery. For example, assume line 34 B is a conical axis line and lines 31 C and 34A are coplanar with line 34B. Lines 34A and 34B are separated by a tilt angle $\phi_1$ and lines 31C and 34A are separated by a tilt angle $\phi_2$. Similarly, lines 31F and 31C are separated by a rotational angle $\theta_1$ and lines 31D and 34C are separated by a rotational angle $\theta_2$.

The internal scan lines are represented by scan lines 34A-C. The number and location of the internal scan lines emanating from the transceiver 10 is variable and may be selected to sufficiently visualize structures within the scan cone 30. The internal scan lines are not peripheral scan lines. The peripheral scan lines 31A-F occupy the conic periphery and converge near the apex of the scan cone 30, thus representing the peripheral limits of the scan cone 30.

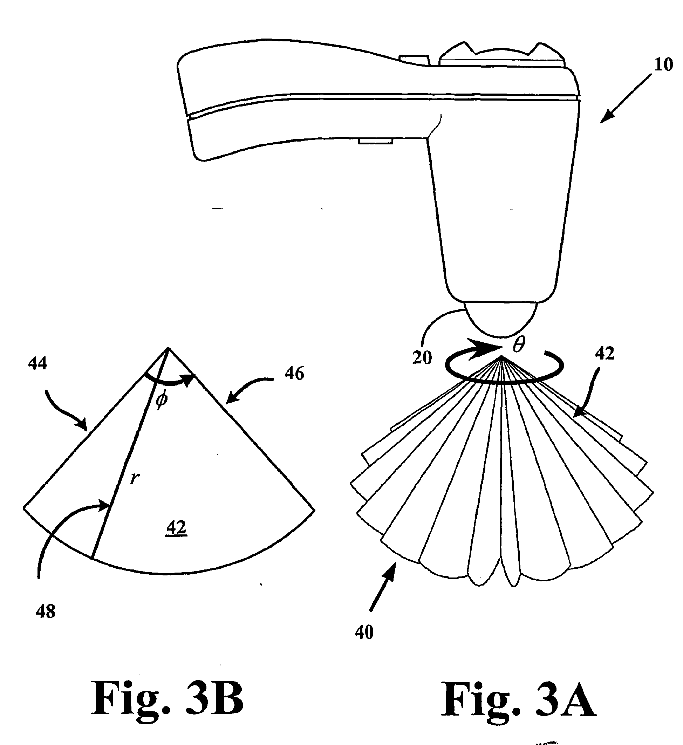

FIGURE 3A is a representation of an ultrasound scan cone emanating from the transceiver in a conic shape formed by a rotational array of two-dimensional scan planes. The scan cone 40 emanating from the dome 20 includes a plurality of scan planes 42 assembled as a rotational array. The scan planes within the rotational array are angularly separated by an angle $\theta$.

FIGURE 3B is a representation of the scan plane of the rotational array. A scan plane 42 is formed by a scan line 48 that rotationally pivots between a first leg 44 and a second leg 46 about a pivot angle $\phi$. The depth of the scan plane 42 is determined by the effective length r of the scan line 48. The area of the scan plane 42 is determined as a product of the length r of the scan line 48 and region swept by the scan line 48 as it migrates about pivot angle $\phi$ between the first and second legs 44 and 46.

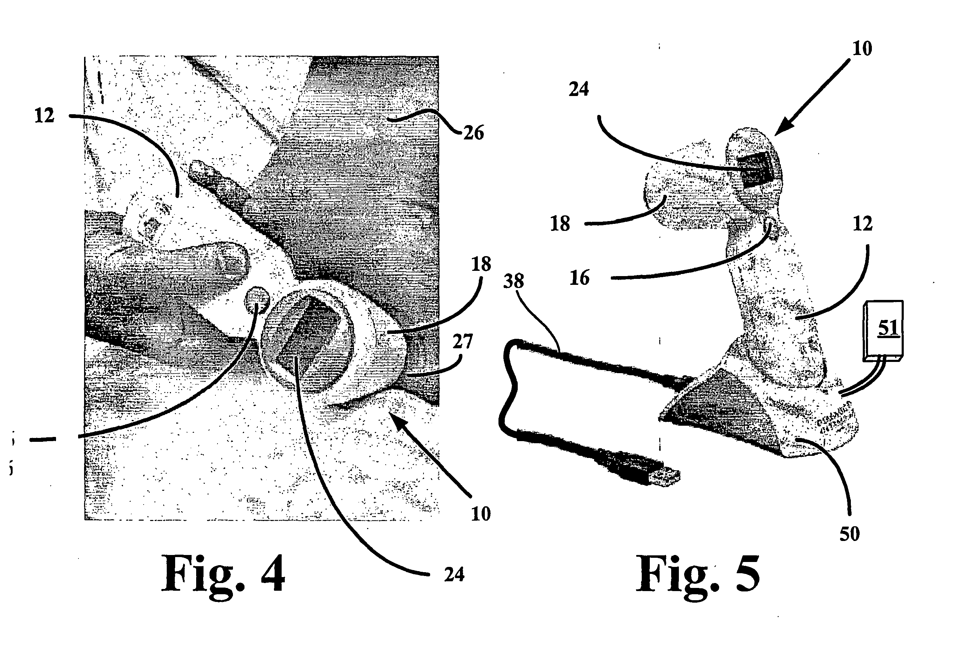

FIGURE 4 is an isometric view of the transceiver 10 positioned on an external portion of a patient 26 that will be used to describe a method of data acquisition for identifying fluids in bodily cavities according to an embodiment of the invention. The transceiver 10 is positioned against a surface portion of the patient 26, and a targeting phase is initiated. In a particular embodiment, the transceiver 10 is then operated in a two-dimensional continuous acquisition mode, which permits data to be continuously acquired and presented as a discrete scan plane image on the display 24 (or another external display device) as the operator physically moves the transceiver 10 across various external portions of the patient 26. In this embodiment, the operator moves the transceiver 10 around an abdominal region and depresses the trigger 14 of the transceiver 10 to continuously acquire real-time two-dimensional images that may be continuously viewed on the display 24, or on another display device. For example, when an anatomical portion that contains a urinary bladder is imaged, urine confined within the bladder appears as a dark region, and a urine fluid area may be calculated. An alphanumeric indication of the urine fluid area (for example, in $cm^2$) may also be calculated and visually presented on the display 24. Similarly, if the patient 26 is a pregnant female, amniotic fluid within the uterus may also be imaged and a corresponding amniotic fluid area may be calculated and displayed on the display 24. After acquisition of the two dimensional measurements, the volume of urine and amniotic fluids are measured in the respective bladder and uterus by acquiring a 3-D scan as a multiple scan plane array similar to the scan cone 40. Alternatively, if the two-dimensional measurements are acquired as three-dimensional distributed scan lines, a three-dimensional scan is accomplished as a three-dimensional scan cone of three-dimensional distributed scan lines similar to the scan cone 30. A cavity selector 16 (as shown in FIGURE 1) is engaged to detect and measure the volumes of either single or multiple cavities in a subject. In a particular embodiment where the transceiver 10 is positioned over the symphasis pubis for acquisition of three-dimensional ultrasound images, a single cavity includes one of the bladder and the uterus, and a multiple cavity- includes the bladder and the uterus.

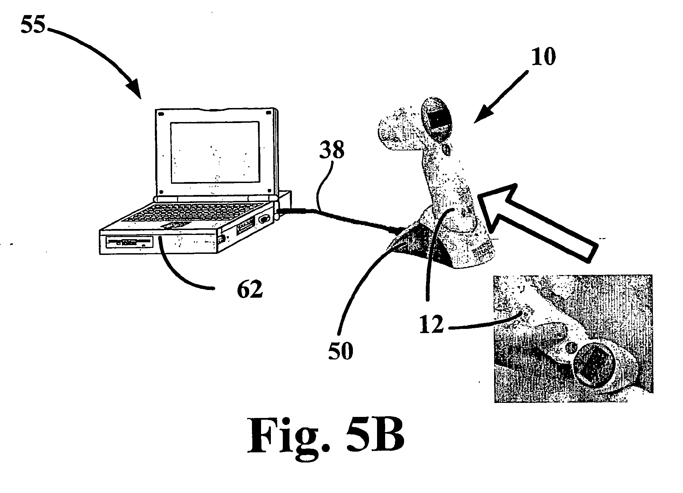

FIGURE 5 is an isometric view of transceiver 10 according to another embodiment of the invention. The transceiver 10 is structured to be received by a support cradle 50. The support cradle 50 is coupled to a power supply 51 that provides electrical energy to the cradle 50 that is communicated to the transceiver 10 either conductively or inductively to provide a charging current to a power supply positioned within the transceiver 10. The support cradle 50 is also configured to receive ultrasound data from the transceiver 10 when positioned in the cradle 50, which may be transferred to an external processor (not shown in FIGURE 3) through a digital communications link 38, such as a link that employs a universal serial bus (USB), a FIREWIRE bus in conformity with IEEE-1394, an RS-232 compatible link, or other similar communications links in conformity with still other protocols. In other embodiments, the communications link 38 may be a wireless link, such as wireless local area network (LAN) or a wireless wide area network (WAN). Alternatively, the cradle 50 may be powered by the link 38.

The communications link 38 may also advantageously provide a means for transferring imaging data from the transceiver 10, and for transferring software updates or software revisions from the external processor to the transceiver 10. In a particular embodiment, the cradle 50 may include a memory device operable to retain digital data received from the transceiver 10 before the data is transferred to the external processor through the communications link 38.

FIGURE 5B illustrates a schematic view of an imaging system 55. The imaging system 55 includes the transceiver 10 positioned in the supporting cradle 50. The communications link 38 connects the transceiver 10 housed in the cradle 12 with a computer 62. The computer 62 may be a desktop, laptop, or other microprocessor-based portable computing device. Data from the transceiver 10 is routed through the cradle 50 to the computer 62 via the communications link 38. The communications link 38 may be a conductive link, as shown in FIGURE 5B, or it may be a wireless radio frequency link or an optical link, such as a wireless infrared link. Within the computer 62 are executable programs to implement the algorithms of the particular embodiments, including the processing of ultrasound signals, retrieving imaging programs, and instructions to perform ultrasound enhancement procedures. Various ultrasound images are developed by processing the ultrasound signal data, including one-dimensional ultrasound images, two-dimensional images, three-dimensional renderings, and enhanced images from the retrieved imaging programs and instructions. The generated images may be stored within the computer 62.

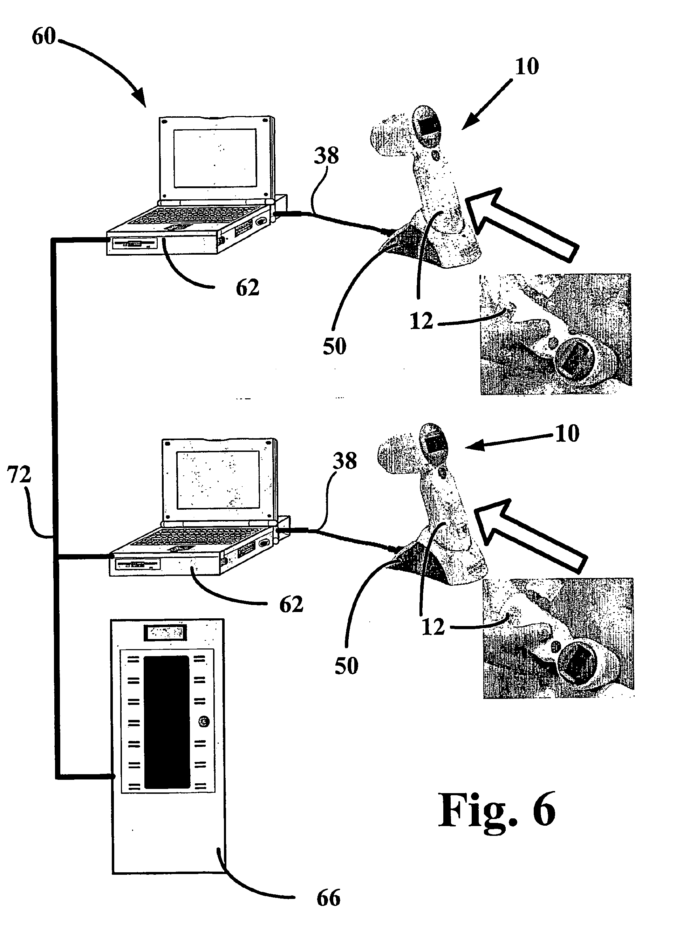

FIGURE 6 is a partial isometric and diagrammatic view of a networked imaging system 60 according to another embodiment of the invention. The imaging system 60 includes one or more transceivers 10 in accordance with one or more of the previously disclosed embodiments. The one or more transceivers 10 may be positioned in supporting cradles 50 that are operably coupled to portable computing devices 62, which in turn, are suitably configured to receive imaging data from the one or more transceivers 10 through the communications link 38. The communications link 38 may be a conductive link, as shown in FIGURE 6, or it may be a wireless radio frequency link or an optical link, such as a wireless infrared link. The portable computing devices 62 communicate with a server 66 over a communications network 72. Although two transceivers 10 are shown in the networked imaging system 60 shown in FIGURE 6, fewer than two, or more than two transceivers 10 may be present. In addition, the processing of ultrasound signals may be divided between the transceiver 10, the portable computing devices 62, and the server 66. For example, the transceiver 10 may be configured to process the ultrasound signals and generate an ultrasound image using algorithms in accordance with other embodiments of the invention, or alternately, the ultrasound image may be generated by the portable computing device 62 or even by the server 66 after receiving ultrasound signals from the transceiver 10. In a particular embodiment of the networked imaging system 60, the imaging algorithms that generate the enhanced ultrasound images reside on the server 66. Each of the portable computing devices 62 accordingly receives signals acquired from the transceivers 10 through the cradles 50 and stores the signals in the portable computing device 62. The computing device 62 subsequently retrieves imaging programs and instructions to perform the additional ultrasound enhancement procedures from the server 66. Thereafter, each personal computing device 62 generates various ultrasound images by processing the ultrasound data, including one-dimensional ultrasound images, two-dimensional images, three-dimensional renderings, and enhanced images from the retrieved imaging programs and instructions. The generated images may be stored on the server 66.

In another particular embodiment, the imaging programs and the instructions reside exclusively on the server 50 and are executed on the server 50. Each portable computing device 62 receives the acquired signals from the transceivers 10 through the cradle 50 and transfers the acquired signals to the portable computing device 62. The device 62 subsequently communicates the signals to the server 66 and processes the signals to generate the desired ultrasound images, including one-dimensional images, two-dimensional images, three-dimensional renderings, and other similar images. The ultrasound images may be stored on the server 66, or alternately, the images may be transferred to one or more of the personal computing devices 62.

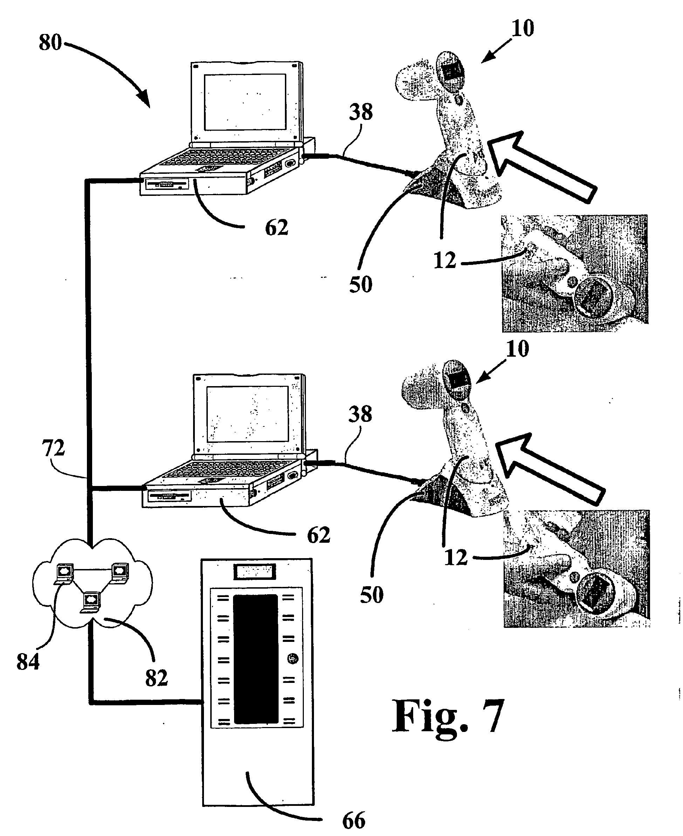

FIGURE 7 is a partial isometric and diagrammatic view of a networked imaging system 80 according to still another embodiment of the invention. Many of the elements of the present embodiment have been discussed in detail in connection with other embodiments, and in the interest of brevity, will not be discussed further. The networked imaging system 80 includes a public data network 82 interposed between the communications network 72 and the server 66. The public data network 82 may include a LAN, a WAN, or the Internet. Accordingly, other computational devices 84 associated with the public data network 82 may communicate imaging data and/or ultrasound images with the portable computing devices 62 and the server 66. Although two transceivers 10 are shown in the networked imaging system 80 shown in FIGURE 7, fewer than two, or more than two transceivers 10 may be present.

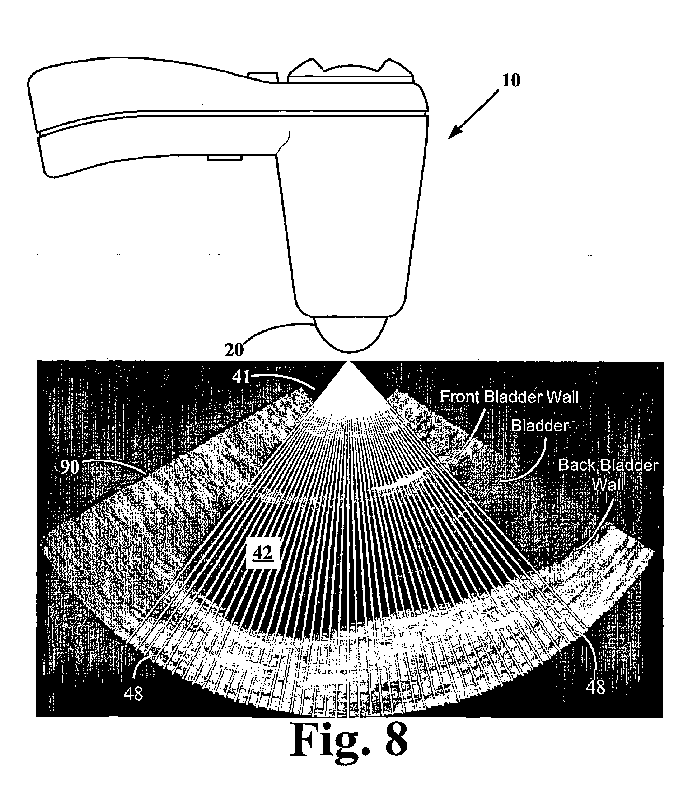

FIGURE 8 is an ultrasound image 90 of a bodily portion of a patient that will be used to describe other embodiments of the invention. The image 90 is formed by projecting a plurality of scan lines 48 downwardly into a selected anatomical portion of the patient to form the fan-like scan plane 42. The scan plane 42 may be rotated about an axis that extends through the transceiver 10 to generate a scan cone 40 (as shown in FIGURE 3A) to obtain three-dimensional imaging information for the selected anatomical portion. Accordingly, when ultrasound energy is projected into the selected anatomical portion, various internal structures may reflect the ultrasound energy, including a bladder, a front bladder wall and a back bladder wall. The bladder may contain a volume of a fluid, such as urine, as shown in FIGURE 8. The foregoing structures typically present imaging resolution difficulties. In particular, an ultrasound image may fail to adequately resolve a fluid-filled cavity, or a tissue that forms a boundary of the fluid-filled cavity, or still other structural details present in the imaged anatomical portion. Moreover, the foregoing structures generally respond to ultrasound energy in a non-linear manner, so that reflected ultrasound echoes include one or more harmonics of a fundamental ultrasound frequency.

One measure of the non-linear behavior of various fluids and tissues is the Goldberg number (G). G is a dimensionless quantity that generally relates ultrasound attenuation to harmonic distortion due to non-linear effects in a tissue or a fluid when subjected to ultrasound energy. Accordingly, when G is about one, non-linear effects are comparable to attenuation effects in the tissue. When G is much greater than one, such as for water or urine, the nonlinear processes are dominant. When G is less than one, as in soft tissue, attenuation effects are more dominant. For example, it is known that fatty tissue has a Goldberg number of approximately about 0.27, while blood, liver, and muscle have a G value of approximately about one. In contrast, fluids such as urine have a G value of approximately about 104.

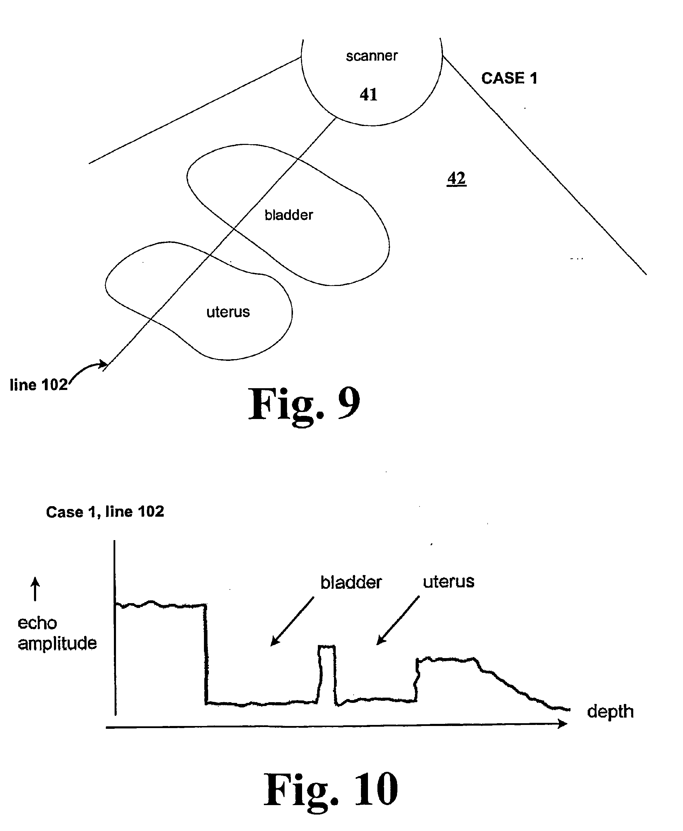

With reference now also to FIGURE 9, the scan plane 42 may include at least one scan line 102 that extends through a bladder and a uterus of a female patient. In this condition, referred to as a "case 1" condition in FIGURE 9; the bladder includes a relatively large volume of urine. Typically, the bladder and the uterus appear as low echogenicity regions when the anatomical region shown in FIGURE 9 is scanned. Known image processing software may be used to image the shallowest region of low echogenicity. Since the low echogenicity region is generally preferentially selected, so that no imaging ambiguity exists, and the bladder is therefore readily identifiable.

Referring now to FIGURE 10, a typical echo amplitude response for the anatomical region of FIGURE 9 is shown. The echo amplitude response as depicted in FIGURE 10 may be obtained through application of one or more of the algorithms. For example, the computational algorithms disclosed in U.S. Pat. No. 6,676,605 to Barnard et al, U.S. Pat. No. 5,235,985 to McMorrow et al, and U.S. Pat. No. 4,926,871 to Ganguly et al, may be used.

When both organs are relatively filled with a fluid (as shown in FIGURE 9), the edges of the bladder and uterus are relatively detectable and are thus generally distinguishable. In this case, the relatively full bladder presents a relatively U-shaped valley at a shallower bodily depth. In contrast, a corresponding U-shaped plateau presented by the uterus is generally identifiable at a greater bodily depth. Thus, while the embodiments of the present invention can improve accuracy and diagnosis in the foregoing situation where both organs are relatively filled with a bodily fluid, the embodiments are also suited to imaging bodily regions when one or both of the organs is less than full.

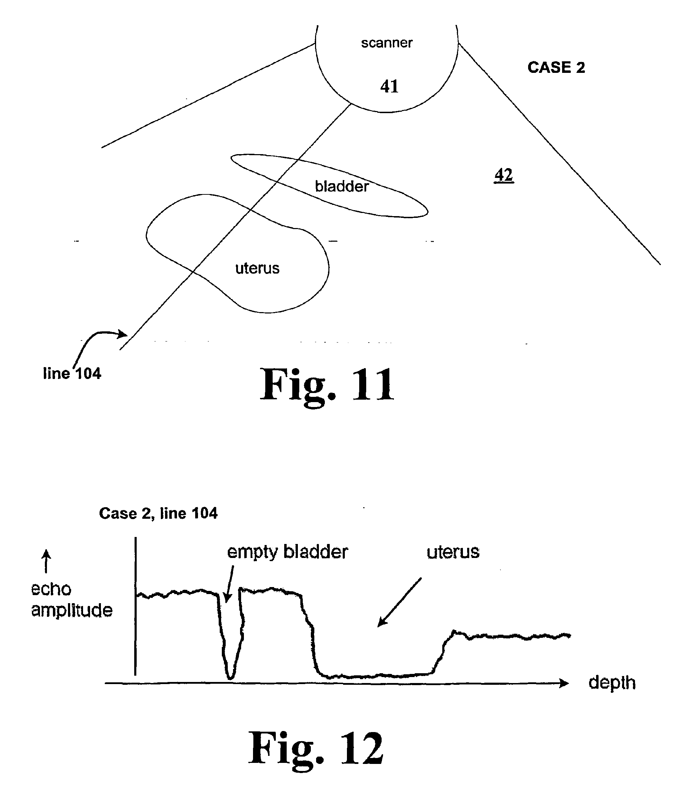

FIGURE 11 is a diagrammatic representation of the anatomical region of FIGURE 9, wherein the at least one scan line 104 extends through the bladder and the uterus when the bladder contains a relatively low volume of urine. This condition is referred to as "case 2" in FIGURE 11. Because the bladder volume is greatly reduced in the Case 2 situation in comparison to the Case 1 condition shown in FIGURE 9, the low echogenicity region is now principally located in the uterus. In current image processing methods, an average of the low echogenicity regions is compared to a threshold value to distinguish between the bladder and the uterus, which may tend to contribute to imaging errors.

FIGURE 12 is an echo amplitude response corresponding to the anatomical region of FIGURE 11. The relatively empty bladder presents a relatively narrow valley. In contrast, the uterus generates a relatively wider U-shaped valley. As a consequence, the bladder is less readily distinguishable from the uterus when the bladder contains a low volume of urine. The disclosed embodiments better address the foregoing problems by using algorithms that more accurately detect ultrasound signal harmonic differences between cavity-residing fluids adjacent to enclosing tissue interfaces.

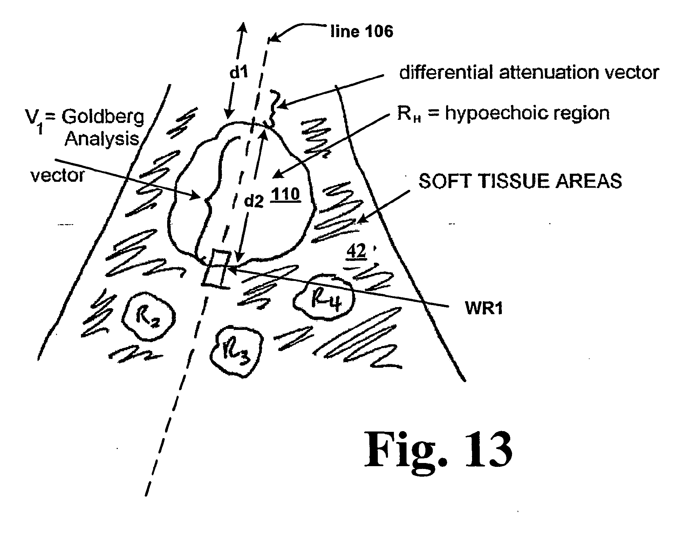

FIGURE 13 is a diagrammatic representation that will be used to illustrate a method for imaging an anatomical region according to an embodiment of the invention. An A-mode scan line 106 is projected into a first bodily cavity 110, for example, a bladder and a uterus as depicted in FIGURES 9 and 11 for the respective case 1 (which corresponds to a relatively full bladder) and case 2 (which corresponds to a nearly empty bladder). The projected ultrasonic waveform is altered by the tissue along the distance $d_1$ of the scan line 106, which corresponds to tissue preceding the cavity 110, and by the distance $d_2$ that corresponds to an interior portion of the cavity 110 along the scan line 106. Other intervening cavities along the scan line 106, such as a uterus, may also alter the projected ultrasound waveform.

The first cavity 110 is hypoechoic and designated as region $R_H$. Other differentially echoic regions illustrated in the scan plane 42 include hypoechoic regions $R_3$, $R_4$, and $R_5$. Ultrasound energy passing through and within the body cavities 110 along scan line 106 may be subjected to an image analysis algorithm to determine respective volumes of each cavity, namely $V_1$ corresponding to the cavity 110. Signals reflected from the back wall or other boundaries of the first body cavity 110 are window function processed in a window region designated as WR1. The WR1 region spans a portion of the tissue adjacent and distal to the cavity 110 backwall interface and further spans a portion of the cavity space along the scan line 106 proximate to the cavity 110-backwall interfaces.

In the WR1 region, window function processing determines the raw data comprising the fundamental frequency $f_o$ and a selected higher order harmonic $2f_o$ that is generated within the WR1 space along the scan line 106. The magnitude of the higher order harmonic generated within WR1 varies because different tissues and/or fluids are encountered by the scan line 106 as it projects into the body. Consequently, a fluid volume and a fluid composition within the cavity 110 alters the magnitude of the higher order harmonic $2f_o$ near the scan line 106 that is proximate to the back wall interface of the cavity 110.

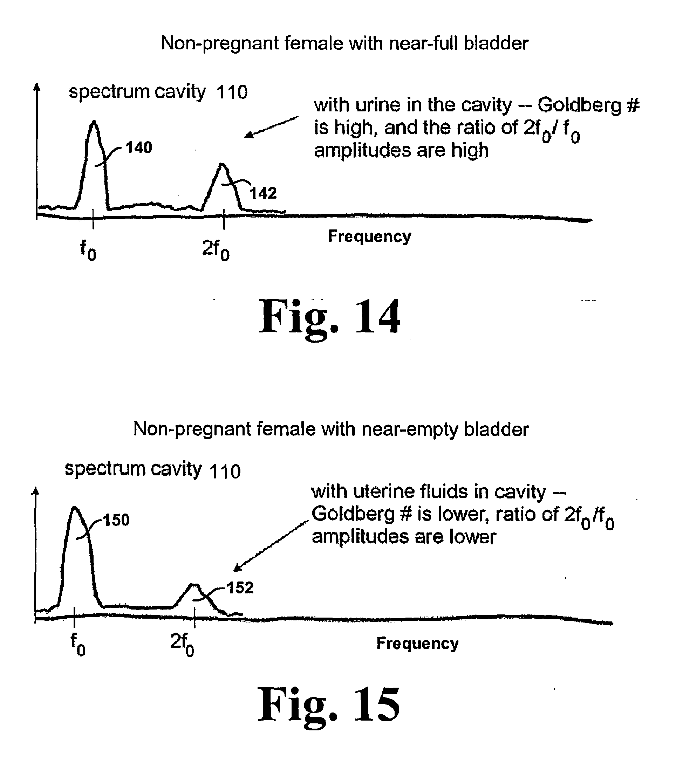

FIGURE 14 is a spectral plot of the insonified region of FIGURE 13 that corresponds to a non-pregnant female with a uterus and nearly full bladder. The fundamental frequency $f_0$ has a peak value 140 and the higher order harmonic $2f_0$ has a peak value 142. The fundamental and harmonic peaks 140 and 142 are a result of window function processing the corresponding echo amplitude response. The magnitude of the harmonic peak 142 may be normalized by dividing the peak 142 by the fundamental frequency peak 140. Accordingly, it is noted that a high Goldberg number stemming from urine in the nearly full bladder corresponds to a high magnitude for the frequency ratio. Different urine volumes and/or the presence of other organs, such as a uterus may also alter the magnitude of the frequency ratio. The magnitude of the second harmonic peak 142 in the first cavity 110 is affected by the presence of the uterus and the urine volume and urine composition contained within the bladder. The composition and volume of the urine may thus be determined.

FIGURE 15 is a spectral plot of the insonified region of FIGURE 13 in a non-pregnant female with a uterus and nearly empty bladder and having greater amounts of blood and tissue containing uterine fluid. The spectral plot is within the window region WR1 and shows the fundamental peak 150 and the higher order harmonic peak 152 resulting from frequency domain processing of the corresponding echo response. As shown,, the harmonic spectrum within WR1 of the non-pregnant female exhibits a generally lower Goldberg number than a non-pregnant female with a substantially greater fluid volume. The magnitude of the frequency ratio $(2f_0/f_0)$ is correspondingly lower. Urine fluids mixed with blood at variable compositions may also alter the magnitude of the higher order harmonic peak 152 shown in FIGURE 15. Accordingly, the reflected spectral components generated within the first cavity 110 may have a still lower harmonic ratio $(2f_0/f_0)$ as compared to unmixed uterine fluids depicted in FIGURE 14, since since the mixed fluid mixtures generally exhibit a lower Goldberg number.

With reference still to FIGURES 14 and 15, an embodiment of the invention will now be described. The present embodiment is based on non-linear wave propagation and variations in the attenuation of ultrasound energy in body fluids. The back wall ultrasound spectrum is processed to determine a reflected harmonic content, and this harmonic content is compared to the content of the fundamental ultrasound energy by forming the frequency ratio. The resulting value may then be adjusted for differences in attenuation at a selected frequency $j$ between $f_0$ and $2f_0$ in the intervening tissue $i$, as described below.

For a selected window, the total attenuation of the fundamental frequency component may be expressed as follows in equation E1:

$$E1: \ A_{f0} = 2d_1 \, \sigma_{11} + 2d_2 \, \sigma_{21} \approx 2d_1 \, \sigma_{11} \qquad dB$$

where: $\sigma_{ij}$ = attenuation coefficient of a tissue $i$ at a frequency $j$ and distances $d_1$ and $d_2$ are as shown in FIGURE 13.

While the total attenuation of the higher order harmonic frequency component may be expressed as follows in equation E2:

$$E2: \quad A_{2f0} = 2d_1 \, \sigma_{12} + 2d_2 \, \sigma_{22} \approx 2d_1 \, \sigma_{12} \quad dB$$

A difference in the attenuation of higher order harmonic component to the fundamental component is therefore defined by equation E3:

$$E3: \quad A_{ratio} = 2d_1 \, (\sigma_{21} - \sigma_{11}) \quad dB.$$

For example, in soft tissue having an attenuation factor of about 1.1 dB/cm, the attenuation coefficient $\sigma_{12}$ is approximately about 3.7 dB/cm , when $f_0$=3.7 MHz. Accordingly, the amplitude ratio becomes in equation E4:

$$E4: \quad A_{ratio} = 2d_1 \, (3.7 \, dB/cm)$$

Based upon the foregoing, harmonic power amplitudes and frequency ratios may be derived and associated with known fluid volumes and fluid compositions derived from living subjects and/or non-living experimental devices, which are then encoded into readily accessible look-up tables, calibration plots or other suitable means for encoding data having utility in measuring the fluid volumes or identifying fluid compositions in an insonified subject.

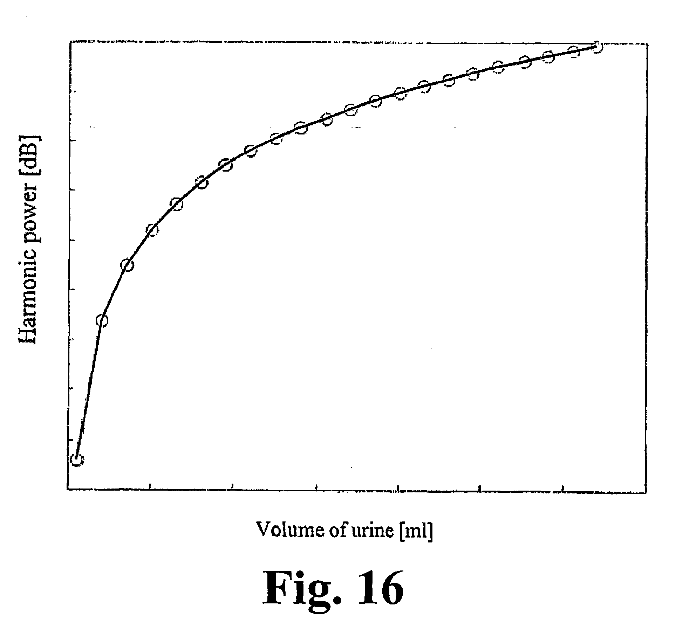

FIGURE 16 is an example of a calibration plot of harmonic power as a function of the bladder volume in a subject. The harmonic power may be obtained from a look-up table that includes data corresponding to different bodily tissues and fluids. The calibration plot thus permits the determination of a urine volume when the higher order amplitude and the fundamental frequency amplitudes are expressed as the ratio $2f_0/f_0$. Alternate embodiments may include calibration plots for other fluid compositions that stem from data in respective look-up tables that are enhanced by the foregoing window functions. For example, a mixture of amniotic fluid and blood, or amniotic fluid/blood/urine mixtures would have a particular calibration plot. The plot shown in FIGURE 16 may alternately be expressed using a suitable curve fitting procedure, such as, for example, a linear least squares procedure, a spline procedure, a polynomial procedure or other known curve fitting procedures.

In still another particular embodiment, the Goldberg number may be used to distinguish between a urinary bladder and a uterus in post-void scans in a female subject. Because the uterus generally appears as a dark structure in ultrasound images, it may be erroneously identified as the urinary bladder in post-void scans. To avoid this, the present embodiment provides that the harmonic amplitudes are calculated on a post-void scan of a female bladder. If a selected combination of harmonic amplitudes is higher than a selected threshold value, the scan likely contains a fluid. Otherwise, the scan likely contains the uterus of the female subject.

In still yet another particular embodiment, the harmonic amplitudes may be calculated based upon one or more selected ultrasound lines, or upon all of the ultrasound lines within the total image. In addition, the harmonics may be calculated within a region-of-interest or lines-of-interest when guided by features detected on the B-mode image, such as a region behind a posterior wall of a fluid-filled cavity.

In still another particular embodiment, inter-patient variability in the harmonic amplitudes may be normalized or otherwise adjusted using a combination of transmission features, such as, for example, frequency, peak-to-peak voltage, pulse length, and other transmission features. Other features may be extracted from B-mode images, such as a depth of an anterior wall of a fluid-filled cavity.

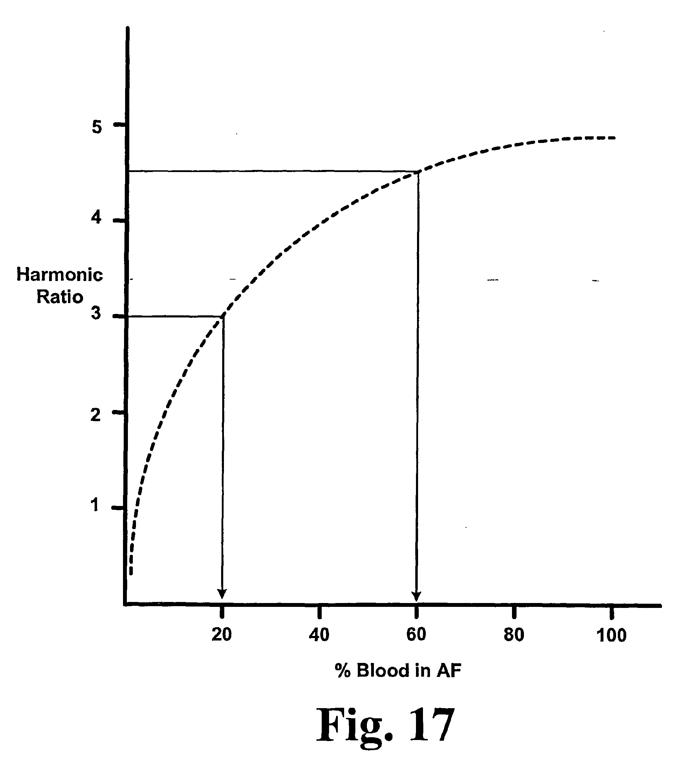

FIGURE 17 is a calibration plot of a harmonic ratio expressed as a function of a blood composition in the amniotic fluid. A dashed calibration line may be used to graphically determine a blood percentage composition. The Goldberg number may also be used to differentiate between various bodily fluid compositions, such as, for example, a transudate and an exudate fluid developed in a lung of a subject, or in compositions of urine. It may also be used to detect the presence of blood within a bodily fluid, such as in amniotic fluid, or in urine. The harmonic ratio differences between the fluids may thus be used to identify a composition of a fluid. For example, as shown in FIGURE 17, a harmonic ratio of 3 corresponds to an amniotic fluid having a blood composition of approximately 20%. Similarly, a harmonic ratio near 4.5 corresponds to an amniotic fluid having approximately 60% blood composition.

In still yet another particular embodiment, the Goldberg number may also be used to distinguish between blood and amniotic fluid in the uterus of a pregnant female. Because blood has a significantly lower Goldberg number compared to amniotic fluid, the second harmonic distortion resulting from a region containing blood is different from a region containing amniotic fluid. The Goldberg number and the harmonic ratio may thus be utilized to differentiate between blood and amniotic fluid and to confirm an identification of the fluid that is isonofied. For example, certain pregnant subjects having a low amniotic fluid volume. Accordingly, the uterus becomes more engorged with blood, making identification of the amniotic fluid regions more difficult. To address this problem, the embodiments of the present invention utilize the Goldberg number and harmonic ratio to assist in the identification process. In a further example, blood appears very dark and similar in appearance to amniotic fluid in a B-mode image. Thus, while measuring an amniotic fluid volume in B-mode images, blood may be detected in the umbilical cord or in the vessels in walls of the uterus and erroneously identified as amniotic fluid. Using the second harmonic distortion in conjunction with the Goldberg Number assists in discriminating between amniotic fluid and blood.

In still another particular embodiment, the Goldberg number may also be used to identify, classify, and measure a volume of a fluid in the lungs for pleural effusion.

In another embodiment, an ultrasound image may be created that shows selected combinations of harmonic amplitudes throughout the ultrasound image that permits detection of various fluid regions that would typically be indicated by presence of higher harmonics. The selected combination of the harmonic reflections may be embodied in a software program, or alternately as an improvement to an existing software program in conventional harmonic imaging ultrasound equipment. The harmonic ratio may then be normalized by the factor, $A_{rati}$. The results may then be compared to an empirically derived look up table or calibration plot that describes one of the attenuation or harmonic characteristics for each kind of tissue (see FIGURE 16). Accordingly, tissues and bodily fluids with low Goldberg numbers and low harmonic ratios can thus be differentiated from tissue and body fluids with higher Goldberg numbers and higher harmonic ratios. The use of the foregoing harmonic ratios and look-up tables (or equivalent calibration plots) in the manner described provides another basis to further differentiate and enhance a display of fluid and tissue regions. For example, a low volume to near empty bladder may be suitably differentiated from a uterine cavity and adjacent tissue.

In another embodiment of the invention, an additional adjustment may be performed to compensate for a "shock formation distance" that may occur along a given scan line. Shock formation distances relate to Goldberg numbers as a consequence of an energy transfer that occurs in the tissues and nearby fluids as the fundamental ultrasound frequency is transformed to a harmonic frequency. For example, an excitation frequency of 3.7 MHz results in significant harmonic generation in human tissue. Thus, the distance $d_2$ (FIGURE 13) may also be used to adjust the harmonic amplitude ratio.

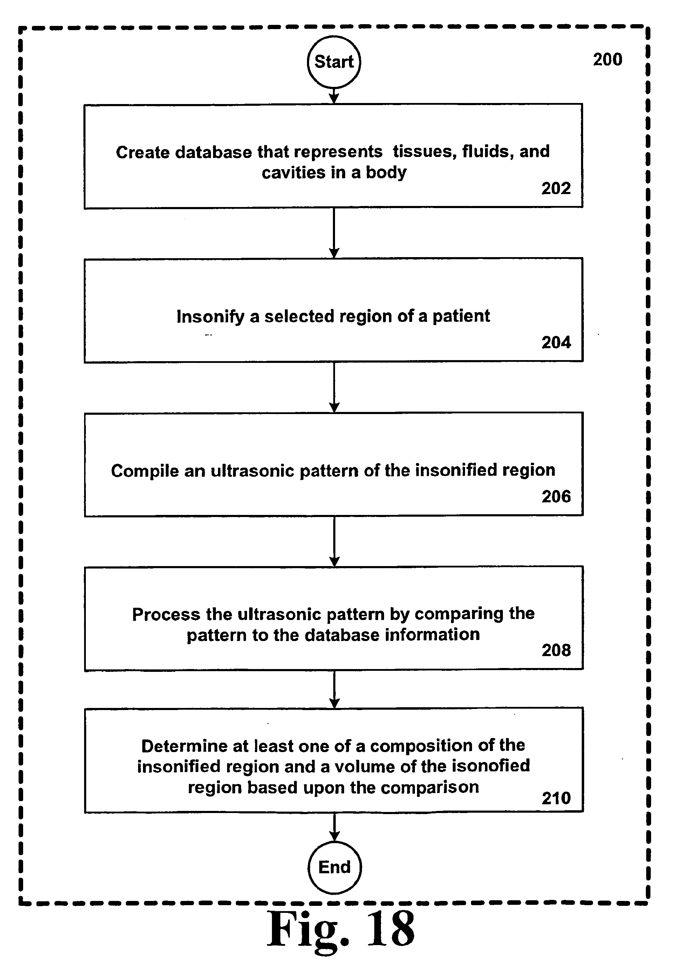

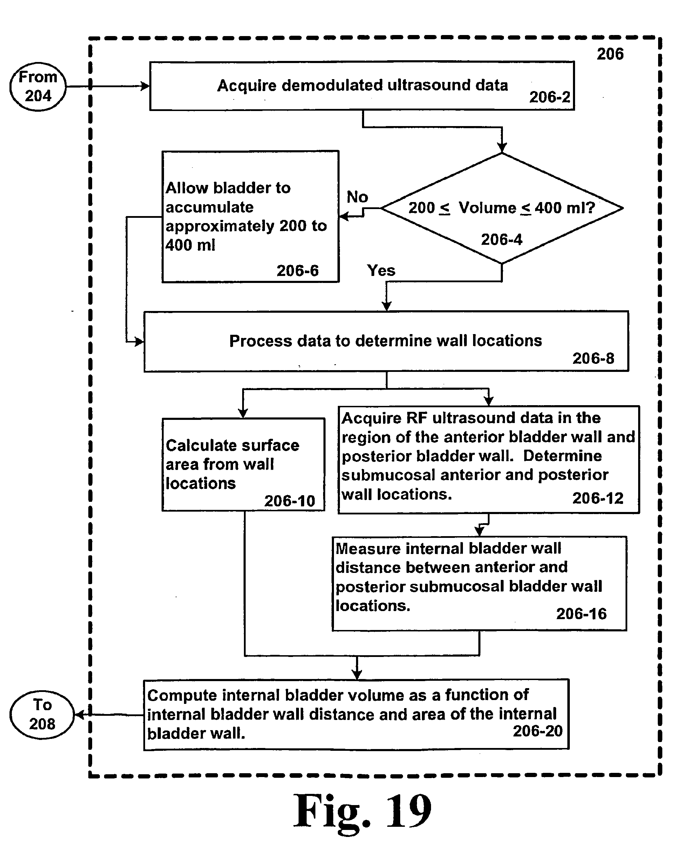

FIGURE 18 illustrates a flow chart of a method 200 of measuring fluid volumes and classifying fluid compositions, according to an embodiment of the invention. The method 200 begins by creating a database that includes attenuation and/or harmonic characteristics of tissues, fluids, and cavities of a body at block 202. The database may be further characterized by sex, age, morphological, physiological, and pathological states. The method 200 continues by isonifying a selected region of a patient at block 204. Thereafter, ultrasonic patterns of the insonified region of the patient are compiled at block 206 (see FIGURE 19 below). The method 200 continues by processing the ultrasonic patterns and comparing the processed patterns to the database information at block 208. For example, the processed patterns may be compared using the volume calibration plot of FIGURE 16 and the composition calibration plot of FIGURE 17. Thereafter, the method 200 concludes by determining at least one of a composition of the insonified region and a volume of the insonified region based upon the comparison of the patient's ultrasonic patterns to the database's content at block 210. Ultrasonic measurement data obtained from the subject at block 210 may then be applied to a volume calibration plot similar to FIGURE 16 to obtain volume measurements. Similarly, ultrasonic measurement data obtained from the subject at block 210 can be applied to a composition calibration plot similar to FIGURE 17 to obtain fluid composition measurements.

Still referring to FIGURE 18, the processed patterns from the subject may be compared to the look-up table or calibration plot as depicted in FIGURE 16 to obtain volume information. To obtain a compositional determination or classification of a given detected fluid, in accord with FIGURE 17, the type of bodily fluids contained within a cavity may be ascertained through a comparative analysis of the Goldberg numbers, harmonic ratios, and attenuation factors within the insonified region. The Goldberg numbers, harmonic ratios, and attenuation factors stored within a database may then be accessed to determine the fluid composition. A fluid composition may thus be determined by accessing a calibration plot, interpolating from a look-up table, or applying regression analysis, as previously described. The volume and compositional look up tables or calibration plots illustrated in FIGURES 16 and 17 may be obtained from ultrasound information databases derived from simulated human models, accumulating clinical measurements obtained from patients stored in a separate database, or a combination of simulated models and clinical measurements. Other databases may include simulated animal models with or without a veterinary-based database. Yet other databases may include a combination of human and veterinary sourced databases.

Similarly, tissue types or combinations thereof within the insonified region are determined by comparative analysis between the Goldberg numbers, harmonic ratios, and attenuation factors presented by the insonified region of the patient to those same numbers, ratios, and factors stored in the database.

FIGURE 19 is an expansion of sub-algorithm 206 of FIGURE 18. Sub-algrothim 206 permits the determination of volume of an organ wall, the mass of an organ wall, the internal organ volume defined by an inner perimeter of an organ wall, and the outer organ volume defined by the outer perimeter of an organ wall from echogenic patterns received from an insonified region. The ultrasonic patterns of the insonified region having at least one organ of the patient are compiled at process block 206-2. Once the wall locations are identified, the wall locations, demodulated magnitude data, and a subset of quadrature amplitude demodulated signals in the region of the anterior bladder wall are directed to the microprocessor for further analysis according to the algorithm illustrated in FIGURE 19 for the particular embodiments. First, ultrasound data is acquired relative to the bladder, uterus, or other organs as shown in the first block 206-2. In general, bladder-specific data can be acquired by a user who manipulates the transceiver 10 while viewing the received data on a display screen and then positioning the transceiver 10 as necessary so that an organ or organs, such as a bladder and uterus, are sufficiently within the field of view of the cone as depicted in FIGURES 2 and 3A.

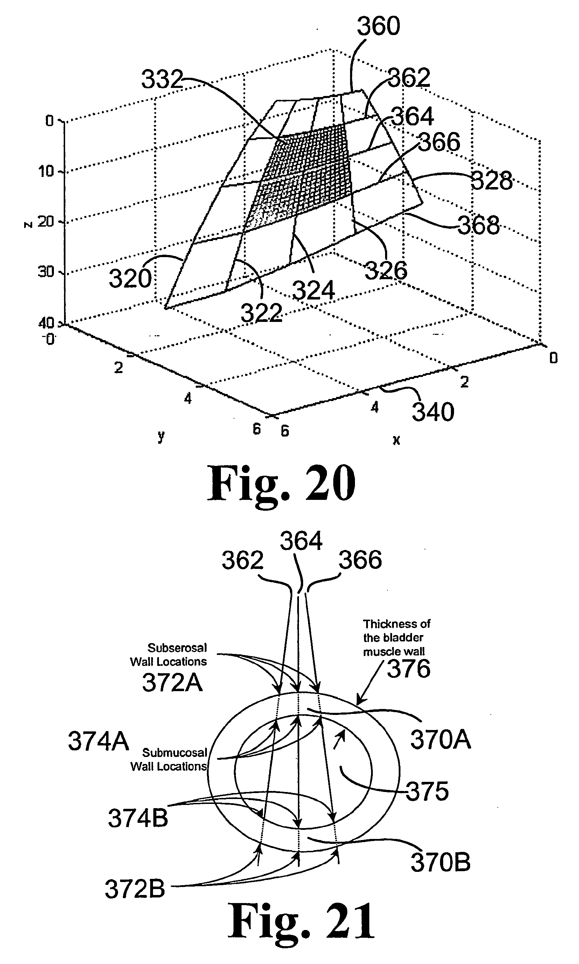

Referring again to FIGURE 19, and limiting the discussion to a specific organ, for example a bladder, echogenic data is collected by the transceiver 10. After obtaining ultrasound bladder data, the ultrasound data is processed to determine if the bladder contains approximately 200 to approximately 400 ml, as shown in the second process block 206-4 represented as a decision diamond. If "No" to the query "200 ml ≤ volume ≤ 400 ml?", then the bladder is allowed to accumulate approximately 200 to approximately 400 ml, as shown in the third process block 206-6, or, if "Yes", meaning the bladder already contains the preferred approximate 200-400 ml volume, then the locations of the bladder walls, as shown in the fourth block 206-8, may be undertaken. The determination of organ wall locations and other such exterior boundaries within an ultrasound scan are within the capability of ultrasound devices presently on the market. In general, however, the process determines the length of a scan line from the transceiver dome to the bladder wall. The data, including wall locations, is stored in the memory of the computer 62 and is used to determine whether or not the bladder volume is within a range of approximately 200 to approximately 400 ml. If the bladder volume is within that range, the ultrasound data is used to determine the actual surface area from the wall locations, as indicated in the fifth block 206-10. The application of previously described methods using harmonic ratios, powers, and Goldberg G-numbers may be used to enhance the accuracy of thickness, area, volume, and mass determinations of bladders holding fluids within the approximate 200-400 ml range. The surface area calculation is explained with regard to FIGURE 21 below and allows for calculation of an outer bladder wall surface area defined by subserosal locations 372A and 372B and an inner bladder wall surface area defined by submucosal locations 374A and 374B. While calculating the surface area in the fifth block 206-10, reflected ultrasound waves are received from the anterior bladder wall, as indicated in the sixth block 206-12. Although these tasks are preferably conducted in parallel, they may alternatively be processed in series. Thereafter, as shown in the seventh block 206-16, the bladder wall thickness is determined from the coherent signals that overlap at the wall locations. The determination of bladder wall thickness is explained in greater detail below. Finally, as shown in the seventh block 206-16, the bladder wall distance is computed as a difference between panterior and posterior submucosal bladder wall locations. Thereafter, at the eighth process block 206-20, the internal bladder volume is computed as a function of the internal bladder wall distances and the area of the internal bladder wall.

The volume restriction indicated in the previous paragraph is included as the range of bladder volumes that allow for an optimal measurement of the bladder wall mass, bladder wall volume, and internal bladder volumes. The volume and mass calculations may be performed at a volume not in this range, but will result in a less accurate measurement that can be corrected by application of the foregoing described methods using harmonic ratios, powers, and Goldberg G-numbers. For example, bladders having less than 200 ml or that are near empty, the foregoing described methods using harmonic ratios, powers, and Goldberg G-numbers will improve the accuracy of determining bladder wall thicknesses, volumes and mass, and internal and outer bladder volumes. For bladders having fluid volumes substantially greater than 400 ml, for example bladder volumes of 1000 ml to multi-liters, the invention will utilize scan lines greater than 20 cm to accommodate the larger bladder sizes. The invention may be applied to measure the thicknesses, masses, and volumes of internal organs of human and animals. The length of the scan lines is adjusted to match the dimension of the internal organ scanned.

The surface area measurement of fifth block 206-4 is performed by integrating the area of interpolating surface patch functions defined by the wall locations. The mathematical calculations are provided below in greater detail.

The surface of the bladder is defined to be $S$. This surface corresponds to the actual surface of the bladder determined by analysis of the wall locations of the bladder. Since this shape is not known in advance, modeling the bladder as a sphere or an ellipsoid provides only a crude approximation of the surface. Instead, the surface $S$ is defined as a construction of a series of individual surface patches $\mathbf{s}_{ij}$, where $i$ and $j$ count through the latitude and longitude components of the surface, similar to the division of the Earth's surface into lines of latitude and longitude. The area of the bladder surface, $S$, is defined as the sum of all the individual surface patches, so that $S = \sum s_{i,j}$.

FIGURE 20 is a schematic representation of four surface patch elements. As depicted in three dimensions in FIGURE 20, by way of example, five scan planes 320-328 are seen transmitted substantially longitudinally across a subserosal wall location 332 referenced to a tri-axis plotting grid 340. The five scan planes include the first scan plane 320, the second scan plane 322, the third scan plane 324, the fourth scan plane 326, and the fifth scan plane 328. The scan planes are represented in the preceding formulas as subscripted variable $j$. Substantially normal to the five longitudinal scan planes are five latitudinal integration lines 360-368 that include a first integration line 360, a second integration line 362, a third integration line 364, a fourth integration line 366, and a fifth integration line 368. The integration lines are represented in the preceding formulas as subscripted variable $i$.

By way of example, four surface patch functions are highlighted in FIGURE 20 as the subserosal wall location 372. The $i$ *and* $j$ subscripts mentioned previously correspond to indices for the lines of latitude and longitude of the bladder surface. For the purposes of this discussion; $i$ will correspond to lines of longitude and $j$ will correspond to lines of latitude although it should be noted the meanings of $i$ and $j$ can be interchanged with a mathematically equivalent result. Using the scan plane and integration line definitions provided in FIGURE 20, the four surface patch functions are identified, in the clockwise direction starting in the upper left, as $s_{322,362}$, $s_{324,362}$, $s_{324,364}$, and $s_{322,364}$.

The surface patches are defined as functions of the patch coordinates, $s_{i,j}(u,v)$. The patch coordinates $u$ and $v$, are defined such that $0 \le u, v < 1$ where 0 represents the starting latitude or longitude coordinate (the $i$ and $j$ locations), and 1 represents the next latitude or longitude coordinate (the $i+1$ and $j+1$ locations). The surface function could also be expressed in Cartesian coordinates where $s_{i,j}(u,v) = x_{ij}(u,v)\mathbf{i} + y_{i,j}(uv)\mathbf{j} + z_{i,j}(u,v)\mathbf{k}$ where $\mathbf{i}, \mathbf{j}, \mathbf{k}$, are unit vectors in the $x$-, $y$-, and $z$- directions respectively. In vector form, the definition of a surface patch function is given in Equation 1. $\mathbf{k}$, are unit vectors in the $x$-, $y$-, and $z$- directions respectively. In vector form, the definition of a surface patch function is given in equation E5.

$$E5: \quad \mathbf{s}_{i,j}(u,v) = \begin{bmatrix} x_{i,j}(u,v) \\ y_{i,j}(u,v) \\ z_{i,j}(u,v) \end{bmatrix}$$

With the definitions of surface patch functions complete, attention can turn to the surface area calculation represented in the fifth block 206-10 of FIGURE 20. The surface area of $\mathbf{S}$, $\mathbf{A(S)}$, can be defined as the integration of an area element over the surface $\mathbf{S}$, as shown in equation E6.

$$E6: \quad A(S) = \int_s dA$$

Since $\mathbf{S}$ is composed of a number of the patch surface functions, the calculation for the area of the surface S can be rewritten as the sum of the areas of the individual surface patch functions as in equation E7.

$$E7: \quad A(S) = \sum_{i,j} A(\mathbf{s}_{i,j}).$$

Similarly to equation E5 for the entire surface, the area of the surface patch is the integration of an area element over the surface patch, shown in equation E8.

$$E8: \quad A(\mathbf{s}_{i,j}) = \int_{s_{i,j}} dA_{i,j}$$

The integration over the surface patch function can be simplified computationally by transforming the integration over the surface to a double integration over the patch coordinates $u$ and $v$. The transformation between the surface integration and the patch coordinate integration is shown in equation E9.

11

$$\text{E9:} \qquad \int_{s_{i,j}} dA_{i,j} = \int_{u=0}^{1} \int_{v=0}^{1} \left| \frac{\partial \mathbf{s}_{i,j}}{\partial u} \times \frac{\partial \mathbf{s}_{i,j}}{\partial v} \right| dv\, du$$

By substituting Equation 5 into Equation 4, and Equation 4 into Equation 3, the area for the entire surface can be calculated. The result of these substitutions is shown in equation E10.

$$\text{E10:} \qquad A(S) = \sum_{i,j} \int_u \int_v \left| \frac{\partial \mathbf{s}_{i,j}}{\partial u} \times \frac{\partial \mathbf{s}_{i,j}}{\partial v} \right| dv\, du$$

The surface patch function may be any function that is continuous in its first derivatives. In the embodiment shown, a cubic B-spline interpolating function is used for the interpolating surface patch function although any surface function may be used. This interpolating function is applied to each of the Cartesian coordinate functions shown in equation E5. The interpolating equation for the x-cooxdinate of the $s_{i,j}$ patch function is given in equation E11.

E11:

$$\text{E11:}$$

$$x_{i,j}(u,v) = \mathbf{u} \mathbf{M}_b \mathbf{X}_{i,j} \mathbf{M}_b^t \mathbf{v}^t$$

where $t$ denotes matrix and vector transpose, $\mathbf{u} = \begin{bmatrix} u^3 \\ u^2 \\ u \\ 1 \end{bmatrix}$, $\mathbf{V} = \begin{bmatrix} v^2 \\ v^2 \\ v \\ 1 \end{bmatrix}$,

$$\mathbf{M}_b = \begin{bmatrix} -1 & 3 & -3 & 1 \\ 3 & -6 & 3 & 0 \\ -3 & 0 & 3 & 0 \\ 1 & 4 & 1 & 0 \end{bmatrix}, \text{ and } \mathbf{X}_{i,j} = \begin{bmatrix} x_{i-1,j-1} & x_{i-1,j} & x_{i-1,j+1} & x_{i-1,j+2} \\ x_{i,j-1} & x_{i,j} & x_{i,j+1} & x_{i,j+2} \\ x_{i+1,j-1} & x_{i+1,j} & x_{i+1,j+1} & x_{i+1,j+2} \\ x_{i+2,j-1} & x_{i+2,j} & x_{i+2,j+1} & x_{i+2,j+2} \end{bmatrix}$$

Similar calculations are performed for the $y_{i,j}$ and $z_{i,j}$ components of the surface patch function.

Since the interpolating functions for each of the patch functions is a cubic surface, the integration may be performed exactly using a quadrature formula. The formula used in this application is shown in equation E12.

$$E12: \quad A(s_{i,j}) = \sum_{i,j} \frac{1}{4} \left( \left| \frac{\partial \mathbf{s}_{i,j}}{\partial u} \times \frac{\partial \mathbf{s}_{i,j}}{\partial v} \right|_{u=\frac{3-\sqrt{3}}{6}, v=\frac{3-\sqrt{3}}{6}} + \left| \frac{\partial \mathbf{s}_{i,j}}{\partial u} \times \frac{\partial \mathbf{s}_{i,j}}{\partial v} \right|_{u=\frac{3-\sqrt{3}}{6}, v=\frac{3+\sqrt{3}}{6}} + \left| \frac{\partial \mathbf{s}_{i,j}}{\partial u} \times \frac{\partial \mathbf{s}_{i,j}}{\partial v} \right|_{u=\frac{3+\sqrt{3}}{6}, v=\frac{3-\sqrt{3}}{6}} + \left| \frac{\partial \mathbf{s}_{i,j}}{\partial u} \times \frac{\partial \mathbf{s}_{i,j}}{\partial v} \right|_{u=\frac{3+\sqrt{3}}{6}, v=\frac{3+\sqrt{3}}{6}} \right)$$

Recalling the fact that $s_{i,j}(u,v)$ is defined as a vector function in Cartesian coordinates (Equation 1), the norm of the cross product of the partial derivatives can be written as follows in equation E13.
$$E13:$$

$$\left| \frac{\partial \mathbf{s}_{i,j}}{\partial u} \times \frac{\partial \mathbf{s}_{i,j}}{\partial u} \right| = \sqrt{ \left( \frac{\partial y_{i,j}}{\partial u} \frac{\partial z_{i,j}}{\partial v} - \frac{\partial z_{i,j}}{\partial u} \frac{\partial y_{i,j}}{\partial v} \right)^2 + \left( \frac{\partial z_{i,j}}{\partial u} \frac{\partial x_{i,j}}{\partial v} - \frac{\partial z_{i,j}}{\partial u} \frac{\partial x_{i,j}}{\partial v} \right)^2 + \left( \frac{\partial x_{i,j}}{\partial u} \frac{\partial y_{i,j}}{\partial v} - \frac{\partial y_{i,j}}{\partial u} \frac{\partial x_{i,j}}{\partial v} \right)^2 }$$

When the physical $x$-, $y$-, and $z$- locations are used in the interpolating function, the surface are will be calculated in the square of the units of $x$, $y$, and $z$. At this point the calculation in the fifth block 206-10 of FIGURE 20 is complete.
The second component to the mass calculation is a measurement of the thickness of the bladder muscle wall. This thickness is defined to be the normal thickness between the subserosal and submucosal surfaces of the bladder wall.
The wall thickness is calculated from the fractal dimension of the RF signal in the region of the wall thickness. The fractal dimension increases due to the multiplicity of interface reflections through the bladder muscle. The increase and decrease of fractal dimension through the bladder muscle wall can be modeled as a parabola where the fractal dimension is a function of the depth in the region of the bladder wall. The thickness of the bladder is then determined to be the region of the parabola model that is at least 97% of the maximal value of the fractal dimension. The calculations are reviewed below in equation E14.
$$E14: \quad fd_r = \frac{\log\left( \frac{\max(RF_{r=r-w/2,r+w/2}) - \min(RF_{r=r-w/2,r+w/2}) + w}{w} \right)}{\log\left( \frac{n}{w} \right)}$$

The wall thickness is calculated from the fractal dimension of the RF signal in the region of the wall thickness. The fractal dimension increases due to the multiplicity of interface reflections through the bladder muscle. The increase and decrease of fractal dimension through the bladder muscle wall can be modeled as a parabola where the fractal dimension is a function of the depth in the region of the bladder wall. The thickness of the bladder is then determined to be the region of the parabola model that is at least 97% of the maximal value of the fractal dimension. The calculations are reviewed below in equation 15.
The fractal dimension calculation corresponds to the fourth block 206-12 of FIGURE 20. The fractal dimension is calculated for a window of length $w$. In the current embodiment the value of $w$ is 5, the number of sample points along a scan line, although that value can be varied. The fractal dimension is calculated from the difference between the maximum RF signal value in the window centered at a given depth, r, and the minimum of that same window. The length of the window, $w$, is added to this difference, and the result is then normalized with the length of the window. The logarithm of that result is then divided by the logarithm of the ratio of the total number of samples in a scan line, *n,* to the length of the window. The calculation of the fractal dimension at each depth along a scan line is shown in Equation 10. This fractal dimension measure is calculated for the central *n-w* samples in a scan line.

After the measurements of the fractal dimension have been calculated based on the ultrasound signal, the thickness of the bladder wall may be calculated. The following calculations correspond to the seventh block 206-16 of FIGURE 19.

The fractal dimension, fd, of the RF signal in the region of the bladder muscle wall is then modeled as a parabolic equation as a function of depth, *r.* The model of the equation for a single depth point is given in equation E15. In that equation, there are 3 parameters (*a, b,* and *c*) that define the parabola with the depth along a scan line r, and the addition of a random element ε The subscript *i* indicates a specific value of *r, fd,* and ε.

$$\text{E15:} \quad fd_i = ar_i^2 + br_i + c + \varepsilon_i$$

An equation of the form in equation E15 is obtained for each depth point in the region of the wall. The number of observations is variable and depends on the thickness of the bladder wall as observed by the ultrasound signal. Assuming a set of *n* observations, the subscript *i* would count the observations from 1 to *n.* The set of *n* equations of the form in equation 15 may be compressed into a matrix equation given in equation E16.

$$\text{E16:}$$

$$\mathbf{fd} = \mathbf{X}\boldsymbol{\beta} + \boldsymbol{\varepsilon}$$

where

$$\mathbf{fd} = \begin{bmatrix} fd_1 \\ fd_2 \\ \vdots \\ fd_n \end{bmatrix}, \mathbf{X} = \begin{bmatrix} r_1^2 & r_1 & 1 \\ r_2^2 & r_2 & 1 \\ \vdots & \vdots & \vdots \\ r_n^2 & r_n & 1 \end{bmatrix}, \boldsymbol{\beta} = \begin{bmatrix} a \\ b \\ c \end{bmatrix}, and \ \boldsymbol{\varepsilon} = \begin{bmatrix} \varepsilon_1 \\ \varepsilon_2 \\ \vdots \\ \varepsilon_n \end{bmatrix}$$

Each row of the **fd,** and ε, and the **X** matrix correspond to one of the *n* observations. The parabola parameters of equation E16 are collected in the vector β.

The next step is to estimate the values of the parameters of the parabola in the set of *n* equations of the form in equation E15 or in the matrix equation E16 based on the set of observations. A least-squares estimation of the parameters is used, and the calculation for these estimates is shown in equation E17. In E17, the *t* superscript indicates matrix transpose, and the -1 superscript indicates the matrix inverse. Parameters with hats (^) indicate that the value is the least-squares estimate of those parameters.

$$\text{E17:} \quad \hat{\boldsymbol{\beta}} = \left(\mathbf{X}'\mathbf{X}\right)^{-1}\mathbf{X}'\mathbf{fd}$$

The estimates of the parabola parameters ( $\hat{\boldsymbol{\beta}} = \begin{vmatrix} \hat{a} & \hat{b} & \hat{c} \end{vmatrix}^t$ ) can be substituted into the parabola model to calculate the estimated fractal dimension at each depth *r*, as shown in equation E18. The location of the maximum fractal dimension can be determined by setting the first derivative of the parabola model to equal 0 (equation E19) and solving for r. The location where the fractal dimension is maximal is given in equation E20.

$$E18: \quad \hat{fd}(r) = \hat{a}r^2 + \hat{b}r + \hat{c}$$

$$E19: \quad \frac{d\hat{fd}(r)}{dr} = 2\hat{a}r + \hat{b} = 0$$

$$E20: \quad r_{fd_{max}} = -\frac{\hat{b}}{2\hat{a}}$$

To determine the maximal fractal dimension as defined by the parabolic model, simply substitute equation 20 into equation 18 and solve for $fd_{max}$. The resulting value is shown in equation E21.

$$E21. \quad \hat{fd}_{max} = \frac{-\hat{b}^2 + 4\hat{c}}{4\hat{a}}$$

To determine the locations where the fractal dimension is 97% of the maximum value, multiply equation E21 by 0.97, substitute the result into equation E18 and solve for r using the quadratic formula. The locations where the fractal dimension is 97% of the maximum value, $r9_{7\%}$, are given in equation E22.

$$E22: \quad r_{97\%} = \frac{-\hat{b} \pm \sqrt{\hat{b}^2 - 4\hat{a}\left(\hat{c} + 0.97\frac{\hat{b}^2 + 4\hat{c}}{4\hat{a}}\right)}}{2\hat{a}}$$

Two values for $r_{97\%}$ will be calculated from Equation 18. The difference between those two values will identify the thickness of the bladder muscle wall along the given scan line. Since these scan lines may or may not be perpendicular to the bladder muscle surface and bladder wall thickness must be measured along a line perpendicular to the bladder surface, a collection of these measurements are combined to determine the actual thickness of the bladder wall.

These measurements could be made at any surface of the bladder muscle wall. In FIGURE 22, three scan lines are shown to cross the bladder muscle in two locations: the anterior wall closest to the transducer, and the posterior wall furthest from the transducer. The parabolic model described previously can be applied twice on each to determine the thickness of both the anterior and posterior wall. The maximum and minimum and mean values of these thicknesses are used in the mass calculation and historical tracking of data. In the embodiment shown, this final thickness determination marks the end of the process identified in the seventh block 206-16 of FIGURE 19.

FIGURE 21 is a schematic representation of three scan lines passing through the subserosal and submucosal wall locations of an organ, here schematically illustrated for a bladder. Three scan lines 362, 364, and 366 penetrate the bladder. The dotted portion of the lines represents the portion of the scan lines that passes through the bladder muscle wall at an anterior or front wall location 370A and a posterior or back wall location 370B. The first 362, the second 364, and the third 366 scan lines are shown transmitting through the front subserosal wall location 372A and front submucosal wall location 374A. Similarly, the first 362, the second 364, and the third 366 scan lines are shown transmitting across the internal bladder region 375 and through the back submucosal wall location 374B and back submserosal wall location 372B. The front and back subserosal locations 372A and 372B occupy an outer bladder wall perimeter and the front and back submucosal locations 374A and 374B occupy an inner bladder wall perimeter. A bladder wall thickness value 376 is obtained for the respective differences along each scan line 362-366 between the subsersosal wall locations 372A and the submucosal wall locations 374A, or the subserosal wall locations 372B and the submucosal wall locations 374B. The maximum and minimum and mean values of these thicknesses are used in the bladder wall mass calculation and historical tracking of data. In the preferred embodiment, the bladder is assumed to have a uniform wall thickness, so that a mean wall thickness value is derived from the scanned data and used for the determination of the internal wall volume 375. Only three scan lines are shown in a plane, each separated by 7.5 degrees from each other. Both the number of scan lines in the plane and the angles separating each scan line within a plane may be varied.

Once the bladder wall thickness and the inner and outer surface area have been measured, the volume of the internal bladder region 375 may be calculated by the determining the respective differences between the front and back submucosal wall locations 374A and 374B along each scanline penetrating the bladder region 375. The difference between the front and back submucosal wall locations 374A and 374B defines an inter-submucosal distance. The internal volume of the bladder region 375 is then calculated as a function of the inter-submucosal distances of the penetrating scan lines and the area of the subserosal boundary or internal bladder perimeter. The volume of internal region 375 is assumed to be the surface area times a function of the inter-submucosal distances, where the assumption is further based on a uniform wall subserosal boundary at all points around the internal bladder perimeter, hi the embodiment shown, this volume calculation corresponds to the eighth block 206-20 of FIGURE 19.

The methods to obtain the wall-thickness data, the mass data, and the volume of internal region 375 via downloaded digital signals can be configured by the microprocessor system for remote operation via the Internet web-based system. The Internet web-based system ("System For Remote Evaluation Of Ultrasound Information Obtained By A Program Application-Specific Data Collection Device") is described in Patent Application Serial # 09/620,766 (Published as US Patent No. 6,569,097). The internet web-based system has multiple programs that collect, analyze, and store organ thickness and organ mass determinations. The alternate embodiment thus provides an ability to measure the rate at which internal organs undergo hypertrophy with time and permits disease tracking, disease progression, and provides educational instructions to patients.

In summary, the foregoing method supplements current algorithms in novel ways that advantageously allow different bodily fluids and/or tissues to be distinguished by volume and composition. In particular, different regions having low echogenicity may be properly distinguished. This feature advantageously permits shadowed regions of a fetus such as the arms and the legs of the fetus to be distinguished from a head region. In a diagnostic method directed to the detection of an aortic aneurysm, the foregoing embodiments may be used to differentiate shadowed regions resulting from bowel gas from other low echo regions. In a gall bladder imaging scan, the foregoing embodiments may be used to determine whether bile or other bodily fluids are within the field of view of the ultrasound-scanning device.

While preferred and alternate embodiments of the invention have been illustrated and described, as noted above, many changes can be made without departing from the scope of the invention. Accordingly, the scope of the invention is not limited by the disclosure of these preferred and alternate embodiments. Instead, the invention should be determined entirely by reference to the claims that follow.

## Claims

1.  An ultrasonography method, comprising
    creating (202) a database that is representative of a fluid of a body;
    transmitting ultrasound pulses into a region-of-interest in a patient along a scan line projected into a patient's cavity containing a patient fluid;
    receiving echoes from the region of interest, and based upon the received echoes:

    determining fundamental and harmonic spectral peaks as a result of window function processing in a window region (WR1) the corresponding echo amplitude response of the signals reflected from the back wall or other boundaries of the patient's body cavity, the window region spanning a portion of the tissue adjacent and distal to the cavity (110) backwall interface and further spanning a portion of the cavity space along the scan line (106) proximate to the cavity (110) backwall interface;
    compiling (206) an ultrasonic pattern of the region-of-interest;

processing (208) the pattern by comparing the region-of-interest patterns to the database; and
determining (210) a composition of the patient fluid within the region of interest of the patient based on the comparison.

2. The method of claim 1, wherein the scan line projects into a cavity containing into at least one of urine, blood, amniotic fluid, lung fluids, liver bile, and mixtures thereof.

3. The method of claim 1 or 2, wherein processing the pattern includes calculating at least one of a Goldberg number, a harmonic ratio, and an attenuation factor.

4. The method of claim 1, comprising transmitting pulses along a plurality of scan lines into the patient and receiving echoes from each scan line from the region of interest.

5. The method of claim 4, comprising performing the subsequent method steps on each scan line.

6. The method of claim 1, further comprising the step of determining the volume of the cavity (110) by image analysis.

7. The method of any preceding claim, wherein the cavity (110) is a bladder or a uterus.

8. An ultrasonography system, comprising:

   a database that is representative of a fluid of a body;
   an ultrasonic apparatus that is configured to transmit ultrasound pulses into a region-of-interest in a patient along a scan line projected into a patient's cavity containing a patient fluid and receive echoes from the region of interest, and based upon the received echoes:

   the system being configured to:

   determine fundamental and harmonic spectral peaks as a result of window function processing in a window (WR1) the corresponding echo amplitude response of the signals reflected from the back wall or other boundaries of the patient's body cavity, the window region spanning a portion of the tissue adjacent and distal to the cavity (110) backwall interface and further spanning a portion of the cavity space along the scan line (106) proximate to the cavity (110) backwall interface;
   compile (206) an ultrasonic pattern of the region-of-interest;
   process (208) the pattern by comparing the region-of-interest patterns to the database; and
   determine (210) a composition of the patient fluid within the region of interest of the patient based on the comparison.

9. The system of claim 8, wherein the scan line projects into a cavity containing into at least one of urine, blood, amniotic fluid, lung fluids, liver bile, and mixtures thereof.

10. The system of claim 8 or 9, wherein process the pattern includes calculate at least one of a Goldberg number, a harmonic ratio, and an attenuation factor.

11. The system of claim 8, wherein the apparatus is configured to transmit pulses along a plurality of scan lines into the patient and to receive echoes from each scan line from the region of interest.

12. The system of claim 4, wherein the system is configured to perform the subsequent steps on each scan line.

13. The system of claim 8, further comprising the apparatus being configured to determine the volume of the cavity (110) by image analysis.

14. The system of any of claims 8 to 13, wherein the cavity (110) is a bladder or a uterus.

**Patentansprüche**

1. Ultraschalluntersuchungsverfahren, das umfasst:

Erzeugen (202) einer Datenbank, die für ein Fluid eines Körpers repräsentativ ist;

Senden von Ultraschallimpulsen in einen interessierenden Bereich eines Patienten längs einer Abtastlinie, die in einen Patientenhohlraum, der einen Patientenfluid enthält, zeigt;

Empfangen von Echos von dem interessierenden Bereich und auf der Grundlage der empfangenden Echos:

Bestimmen spektraler Grund-Peaks und harmonischer Peaks als ein Ergebnis einer Fensterfunktionsverarbeitung in einem Fensterbereich (WR1) der entsprechenden Echoamplitudenantwort der Signale, die von der Rückwand oder von anderen Grenzen des Körperhohlraums des Patienten reflektiert werden, wobei der Fensterbereich einen Abschnitt des Gewebes angrenzend an die und distal zu der Rückwandgrenzfläche des Hohlraums (110) umfasst und ferner einen Abschnitt des Hohlraums längs der Abtastlinie (106) proximal zu der Rückwandgrenzfläche des Hohlraums (110) umfasst;

Kompilieren (206) eines Ultraschallmusters des interessierenden Bereichs;

Verarbeiten (208) des Musters durch Vergleichen der Muster des interessierenden Bereich mit der Datenbank; und

Bestimmen (210) einer Zusammensetzung des Patientenfluids in dem interessierenden Bereich des Patienten auf der Grundlage des Vergleichs.

2. Verfahren nach Anspruch 1, wobei die Abtastlinie in einen Hohlraum zeigt, der wenigstens eines der folgenden Fluide enthält: Urin, Blut, Fruchtwasser, Lungenfluide, Lebergalle und Gemische hiervon.

3. Verfahren nach Anspruch 1 oder 2, wobei das Verarbeiten des Musters das Berechnen einer Goldberg-Nummer und/oder eines harmonischen Verhältnisses und/oder eines Dämpfungsfaktors umfasst.

4. Verfahren nach Anspruch 1, das das Senden von Impulsen längs mehrerer Abtastlinien in den Patienten und das Empfangen von Echos von jeder Abtastlinie von dem interessierenden Bereich umfasst.

5. Verfahren nach Anspruch 4, das das Ausführen der späteren Verfahrensschritte auf jeder Abtastlinie umfasst.

6. Verfahren nach Anspruch 1, das ferner den Schritt des Bestimmens des Volumens des Hohlraums (110) durch Bildanalyse umfasst.

7. Verfahren nach einem vorhergehenden Anspruch, wobei der Hohlraum (110) eine Blase oder eine Gebärmutter ist.

8. Ultraschalluntersuchungssystem, das umfasst:

eine Datenbank, die für ein Fluid eines Körpers repräsentativ ist;

eine Ultraschallvorrichtung, die konfiguriert ist, um Ultraschallimpulse in einen interessierenden Bereich eines Patienten längs einer Abtastlinie zu senden, die in einen Patientenhohlraum, der ein Patientenfluid enthält, zeigt, und um Echos von dem interessierenden Bereich zu empfangen, wobei auf der Grundlage der empfangenen Echos:

das System konfiguriert ist, um:

spektrale Grund-Peaks und harmonische Peaks als ein Ergebnis einer Fensterfunktionsverarbeitung in einem Fenster (WR1) der entsprechenden Echoamplitudenantwort der Signale, die von der Rückwand oder von anderen Grenzen des Körperhohlraums des Patienten reflektiert werden, zu bestimmen, wobei der Fensterbereich einen Abschnitt des Gewebes angrenzend an die und distal zu der Rückwandgrenzfläche des Hohlraums (110) umfasst und ferner einen Abschnitt des Hohlraums längs der Abtastlinie (106) proximal zu der Rückwandgrenzfläche des Hohlraums (110) umfasst;

ein Ultraschallmuster des interessierenden Bereiches zu kompilieren (206);

das Muster durch Vergleichen der Muster des interessierenden Bereichs mit der Datenbank zu verarbeiten (208); und

eine Zusammensetzung des Patientenfluids in dem interessierenden Bereich des Patienten auf der Grundlage des Vergleichs zu bestimmen (210).

9. System nach Anspruch 8, wobei die Abtastlinie in einen Hohlraum zeigt, der wenigstens eines der folgenden Fluide enthält: Urin, Blut, Fruchtwasser, Lungenfluide, Lebergalle und Gemische hiervon.

**10.** System nach Anspruch 8 oder 9, wobei das Verarbeiten des Musters das Berechnen einer Goldberg-Nummer und/oder eines harmonischen Verhältnisses und/oder eines Dämpfungsfaktors umfasst.

**11.** System nach Anspruch 8, wobei die Vorrichtung konfiguriert ist, um Impulse längs mehrerer Abtastlinien in den Patienten zu senden und um Echos von jeder Abtastlinie von dem interessierenden Bereich zu empfangen.

**12.** System nach Anspruch 4, wobei das System konfiguriert ist, um die späteren Schritte auf jeder Abtastlinie auszuführen.

**13.** System nach Anspruch 8, das ferner umfasst, dass die Vorrichtung konfiguriert ist, um das Volumen des Hohlraums (110) durch Bildanalyse zu bestimmen.

**14.** System nach einem der Ansprüche 8 bis 13, wobei der Hohlraum (110) eine Blase oder eine Gebärmutter ist.

**Revendications**

**1.** Procédé d'échographie, consistant à :

créer (202) une base de données qui est représentative d'un fluide corporel ;
transmettre des impulsions ultrasonores dans une région d'intérêt chez un patient suivant une ligne de balayage projetée à l'intérieur d'une cavité du patient contenant un fluide du patient ;
recevoir des échos de la région d'intérêt et, sur la base des échos reçus :

déterminer des pics spectraux fondamentaux et harmoniques comme résultat d'un traitement par une fonction de fenêtre, dans une région de fenêtre (WR1), de la réponse en amplitude d'écho correspondante des signaux réfléchis par la paroi arrière ou par d'autres frontières de la cavité corporelle du patient, la région de fenêtre couvrant une partie des tissus adjacents et distaux par rapport à l'interface de la paroi arrière de la cavité (110) et couvrant en outre une partie de l'espace de la cavité suivant la ligne de balayage (106) à proximité de l'interface avec la paroi arrière de la cavité (110) ;
compiler (206) un motif ultrasonore de la région d'intérêt ;
traiter (208) le motif en comparant les motifs de régions d'intérêt à la base de données ; et
déterminer (210) une composition du fluide du patient à l'intérieur de la région d'intérêt du patient sur la base de la comparaison.

**2.** Procédé selon la revendication 1, dans lequel la ligne de balayage se projette à l'intérieur d'une cavité renfermant au moins l'un des fluides suivantes :

de l'urine, du sang, du fluide amniotique, des fluides pulmonaires, de la bile et des mélanges de ceux-ci.

**3.** Procédé selon la revendication 1 ou 2, dans lequel le traitement du motif consiste à calculer au moins l'un d'un nombre de Goldberg, d'un rapport d'harmoniques et d'un facteur d'atténuation.

**4.** Procédé selon la revendication 1, consistant à émettre des impulsions suivant une pluralité de lignes de balayage à l'intérieur du patient et à recevoir des échos en provenance de chaque ligne de balayage de la région d'intérêt.

**5.** Procédé selon la revendication 4, consistant à effectuer les étapes de procédé consécutives sur chaque ligne de balayage.

**6.** Procédé selon la revendication 1, comprenant en outre l'étape consistant à déterminer le volume de la cavité (110) par analyse d'image.

**7.** Procédé selon l'une quelconque des revendications précédentes, dans lequel la cavité (110) est une vessie ou un utérus.

**8.** Système d'échographie, comprenant :

une base de données qui est représentative d'un fluide corporel ;

un appareil à ultrasons qui est configuré pour émettre des impulsions ultrasonores à l'intérieur d'une région d'intérêt chez un patient suivant une ligne de balayage projetée à l'intérieur d'une cavité du patient contenant un fluide du patient et recevoir des échos de la région d'intérêt et, sur la base des échos reçus :

le système étant configuré pour :

déterminer des pics spectraux fondamentaux et harmoniques comme résultat d'un traitement par une fonction de fenêtre, dans une fenêtre (WR1), de la réponse en amplitude d'écho correspondante des signaux réfléchis par la paroi arrière ou d'autres frontières de la cavité corporelle du patient, la région de fenêtre couvrant une partie des tissus adjacents et

distaux par rapport à l'interface de la paroi arrière de la cavité (110) et couvrant en outre une partie de l'espace de la cavité suivant la ligne de balayage (106) à proximité de l'interface avec la paroi arrière de la cavité (110) ;

compiler (206) un motif ultrasonore de la région d'intérêt ;

traiter (208) le motif en comparant les motifs de régions d'intérêt à la base de données ; et

déterminer (210) une composition du fluide du patient à l'intérieur de la région d'intérêt du patient sur la base de la comparaison.

**9.** Système selon la revendication 8, dans lequel la ligne de balayage se projette à l'intérieur d'une cavité renfermant au moins l'un des fluides suivantes : de l'urine, du sang, du fluide amniotique, des fluides pulmonaires, de la bile et des mélanges de ceux-ci.

**10.** Système selon la revendication 8 ou 9, dans lequel le traitement du motif consiste à calculer au moins l'un d'un nombre de Goldberg, d'un rapport d'harmoniques et d'un facteur d'atténuation.

**11.** Système selon la revendication 8, dans lequel l'appareil est configuré pour émettre des impulsions suivant une pluralité de lignes de balayage à l'intérieur du patient et pour recevoir des échos en provenance de chaque ligne de balayage de la région d'intérêt.

**12.** Système selon la revendication 4, dans lequel le système est configuré pour effectuer les étapes consécutives sur chaque ligne de balayage.

**13.** Système selon la revendication 8, dans lequel l'appareil est en outre configuré pour déterminer le volume de la cavité (110) par analyse d'image.

**14.** Système selon l'une quelconque des revendications 8 à 13, dans lequel la cavité (110) est une vessie ou un utérus.

**FIG. 1**

**Fig. 2**

**Fig. 3B**

**Fig. 3A**

Fig. 5

Fig. 4

Fig. 5B

**Fig. 6**

Fig. 7

**Fig. 8**

**Fig. 9**

Case 1, line 102

**Fig. 10**

scanner

41

CASE 2

42

bladder

uterus

line 104

**Fig. 11**

Case 2, line 104

empty bladder

uterus

echo
amplitude

depth

**Fig. 12**

Fig. 13

Non-pregnant female with near-full bladder

spectrum cavity 110

with urine in the cavity -- Goldberg # is high, and the ratio of $2f_0/f_0$ amplitudes are high

140

142

$f_0$      $2f_0$      **Frequency**

# Fig. 14

Non-pregnant female with near-empty bladder

spectrum cavity 110

with uterine fluids in cavity -- Goldberg # is lower, ratio of $2f_0/f_0$ amplitudes are lower

150

152

$f_0$      $2f_0$      **Frequency**

# Fig. 15

Volume of urine [ml]

# Fig. 16

**% Blood in AF**

# Fig. 17

Start

200

Create database that represents tissues, fluids, and cavities in a body

202

Insonify a selected region of a patient

204

Compile an ultrasonic pattern of the insonified region

206

Process the ultrasonic pattern by comparing the pattern to the database information

208

Determine at least one of a composition of the insonified region and a volume of the isonofied region based upon the comparison

210

End

## Fig. 18

206

From
204

Acquire demodulated ultrasound data
206-2

Allow bladder to
accumulate
approximately 200 to
400 ml
206-6

No

200 ≤ Volume ≤ 400 ml?
206-4

Yes

Process data to determine wall locations
206-8

Calculate surface
area from wall
locations
206-10

Acquire RF ultrasound data in the
region of the anterior bladder wall and
posterior bladder wall. Determine
submucosal anterior and posterior
wall locations.
206-12

Measure internal bladder wall
distance between anterior and
posterior submucosal bladder wall
locations.
206-16

To
208

Compute internal bladder volume as a function of
internal bladder wall distance and area of the internal
bladder wall.
206-20

# Fig. 19

**Fig. 20**

**Fig. 21**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6676605 B, Barnard
- US 5235985 A, McMorrow
- US 4926871 A, Ganguly
- US 09620766 B
- US 6569097 B

### Non-patent literature cited in the description

- **A. Bouakaz ; E. Merks ; C. Lancee ; N. Bom.** Noninvasive Bladder Volume Measurements Based on Nonlinear Wave Distortions. *Ultrasound in Medicine & Biology,* vol. 30 (4), 469-476